# EUROPÄISCHE PATENTANMELDUNG

(11) **EP 2 308 460 A1**
(43) Veröffentlichungstag der Anmeldung: **13.04.2011**
(21) Anmeldenummer: 10184153.4
(22) Anmeldetag: 20.02.2006
(51) Int. Cl.: A61K 8/84, A61K 8/87, A61K 8/88, A61K 8/85, A61Q 5/10, A61Q 5/08, A61Q 1/02, A61Q 19/04, A61Q 19/02, A61Q 17/04, A61Q 17/02, A61Q 17/00, A61Q 5/00, A61Q 19/00

(54) **Wirkstoffzusammensetzung die wenigstens ein stickstoffatomhaltiges, hyperverzweigtes Polymer enthält**

(30) Priorität: 21.02.2005 DE 102005007844
(62) Teilanmeldung aus: 06707095.3
(71) Anmelder: BASF SE, 67056 Ludwigshafen (DE)
(72) Erfinder: Bouillo, Nathalie, 76532, Baden-Baden (DE); Krüger, Christian, 55291, Saulheim (DE); Pierobon, Marianna, 67063, Ludwigshafen (DE); Bruchmann, Bernd, 67251, Freinsheim (DE); Stumbe, Jean-Francois, F-67200, Strasbourg (FR); Lange, Ronald, Frans, Maria, 67061, Ludwigshafen (DE)
(74) Vertreter: Reitstötter - Kinzebach

(57) **Zusammenfassung**

Die vorliegende Erfindung betrifft eine Wirk- oder Effektstoffzusammensetzung, die wenigstens einen in Wasser schwerlöslichen Wirk- oder Effektstoff und wenigstens ein hyperverzweigtes Polymer enthält.

## Beschreibung

Die vorliegende Erfindung betrifft eine Wirk- oder Effektstoffzusammensetzung, die wenigstens einen in Wasser schwerlöslichen Wirk- oder Effektstoff und wenigstens ein stickstoffatomhaltiges, hyperverzweigtes Polymer enthält.

Wirkstoffe für Arzneimittel, den Pflanzenschutz, die Kosmetik und für den Materialschutz, d. h. Substanzen, die auch in geringer Konzentration bereits eine Wirkung entfalten, z.B. eine pharmakologische Wirkung in einem Organismus, eine physiologische Wirkung in einer Pflanze oder einem Schadorganismus, eine kosmetische Wirkung, etc., werden häufig in Form wässriger Wirkstoffzubereitungen formuliert und angewendet. Alternativ ist auch eine Formulierung und Verabreichung in fester Form, z. B. als Pulver oder Pressling (Tablette, etc.) möglich, wobei der Transport an den eigentlichen Wirkort jedoch die Umwandlung in eine wässrige Form umfasst.

Ein prinzipielles Problem bei wässrigen Wirkstoffzubereitungen ist die geringe Wasserlöslichkeit vieler Wirkstoffe, die häufig weniger als 5 g/l bei 23 °C/1013 mbar beträgt. Wässrige Formulierungen derartiger Wirkstoffe können als heterogene Systeme vorliegen, wobei der Wirkstoff als emulgierte bzw. dispergierte Phase in einer kontinuierlichen wässrigen Phase vorliegt. Zur Stabilisierung dieser an sich metastabilen Systeme werden üblicherweise Emulgatoren oder Dispergierhilfsmittel eingesetzt. Deren stabilisierende Wirkung ist jedoch häufig nicht zufriedenstellend, so dass eine Abscheidung des Wirkstoffs, beispielsweise ein Aufrahmen oder ein Sedimentieren des Wirkstoffs, auftreten kann, insbesondere, wenn die wässrige Formulierung längere Zeit bei erhöhter Temperatur und/oder bei stark wechselnden Temperaturen oder in der Nähe des Gefrierpunkts gelagert wird. Dieses Problem ist insbesondere dann ausgeprägt, wenn der Wirkstoff zur Kristallisation neigt. Zudem wird vielfach eine Solubilisierung, d. h. eine Löslichkeitsverbesserung durch oberflächenaktive Verbindungen, angestrebt, die die schlecht wasserlöslichen oder wasserunlöslichen Stoffe in klare, höchstens opaleszierende wässrige Lösungen überführt, ohne dass hierbei die chemische Struktur dieser Stoffe eine Veränderung erfährt. Diese Solubilisate sind dadurch gekennzeichnet, dass der schlecht wasserlösliche oder wasserunlösliche Stoff in den Molekülassoziaten der oberflächenaktiven Verbindungen, die sich in wässriger Lösung bilden gelöst vorliegt. Die resultierenden Lösungen sind stabile einphasige Systeme, die optisch klar bis opaleszent erscheinen und ohne Energieeintrag hergestellt werden können. Solubilisatoren können beispielsweise das Aussehen von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Häufig werden auch organische Lösungsmittel zur Herstellung wässriger Formulierungen von in Wasser nicht löslichen Wirkstoffen eingesetzt. So verwendet man häufig mit Wasser mischbare Lösungsmittel als Löslichkeitsvermittler, d. h. zur Erhöhung der Löslichkeit des Wirkstoffs in der wässrigen Phase. Mit Wasser nicht mischbare Lösungsmittel wiederum dienen dazu, einen bei Anwendungstemperatur festen Wirkstoff in eine flüssige Phase zu überführen, die man dann emulgieren kann. Im Unterschied zum festen Wirkstoff ist der Wirkstoff über das Lösemittel in der Emulsion molekular gelöst und bei Applikation besser verfügbar und wirksamer. Der Einsatz organischer Lösungsmittel ist jedoch auf Grund der bekannten VOC-Problematik aus arbeitshygienischen Gründen, Umweltaspekten und teilweise auch aus toxikologischen Gründen nicht wünschenswert.

Verschiedentlich wurde vorgeschlagen, wasserunlösliche Wirkstoffe in Form wässriger Mikro- bzw. Nanoemulsionen zu formulieren. Allerdings benötigt man zur Herstellung derartiger Mikro- bzw. Nanoemulsionen vergleichsweise große Mengen an Emulgator und an organischen Lösungsmitteln. Der hohe Anteil an Emulgatoren ist jedoch nicht nur ein Kostenfaktor sondern kann auch zu Problemen bei der Anwendung der Formulierungen führen. Lösungsmittel wiederum sind aus arbeitshygienischen Gründen und Kostengründen unerwünscht. Ein weiteres Problem derartiger Mikroemulsionen ist ihre Instabilität gegenüber Entmischung.

Weiterhin wurden verschiedentlich wässrige Polymer/Wirkstoffzubereitungen beschrieben, die durch radikalische, wässrige Emulsionspolymerisation einer Monomeremulsion erhalten werden, worin der Wirkstoff in den Monomertröpfchen der zu polymerisierenden Monomeremulsion vorliegt. Dieses Verfahren ist jedoch auf solche Wirkstoffe beschränkt, die in den Monomeren gut löslich sind. In der Regel handelt es sich um Substanzen, die bei Raumtemperatur flüssig sind.

Verschiedentlich wurde auch die Verwendung von amphiphilen Copolymeren zur Solubilisierung von in Wasser unlöslichen Wirkstoffen in einem wässrigen Vehikel vorgeschlagen. So schlägt beispielsweise die US 2003/0009004 zu diesem Zweck amphiphile Blockcopolymere vor, die einen hydrophilen Polyethylenimin-Block und einen hydrophoben Block aus einem biologisch abbaubaren, aliphatischen Polyester umfassen.

Die US 2003/0157170 beschreibt wasserfreie Wirkstoffzusammensetzungen, die ein amphiphilen Diblockcopolymer mit einem Polyester als hydrophobem Bestandteil und ein Additiv enthalten. Die Zusammensetzungen bilden beim Verdünnen mit Wasser wirkstoffhaltige Mizellen.

Die WO 02/82900 beschreibt die Verwendung amphiphiler Blockcopolymere zur Herstellung wässriger Suspensionen von wasserunlöslichen Pflanzenschutzwirkstoffen. Die eingesetzten Blockcopolymere sind erhältlich durch "lebende" bzw. "kontrollierte" radikalische Blockcopolymerisation ethylenisch ungesättigter Monomere.

Die US 4,888,389 beschreibt Blockcopolymere, die einen Polyisobutenblock und einen hydrophilen Block, beispielsweise einen Polyetherblock, aufweisen.

Die unveröffentlichte deutsche Patentanmeldung 10 2004 027 835.0 beschreibt die Verwendung amphiphiler Polymerzusammensetzungen, die durch Umsetzung mit Polyisocyanaten verknüpfte Blöcke von hydrophilen und hydrophoben Polymeren aufweisen, zur Herstellung wässriger Formulierungen von nicht oder nur gering wasserlöslichen Wirkstoffen und Effektstoffen.

Auch statistische, amphiphile Copolymere wurden als Solubilisatoren angewendet. So betrifft die EP-A 0 876 819 die Verwendung von Copolymerisaten aus N-VinylPyrrolidon und Alkylacrylsäuren als Solubilisatoren.

Die EP-A 0 953 347 betrifft die Verwendung von Polyalkylenoxid-haltigen Pfropfpolymerisaten als Solubilisatoren.

Aus der EP-A 0 943 340 ist die Verwendung von polymerisierten Fettsäurederivaten und Fettalkoholderivaten als Solubilisatoren bekannt.

Die EP-A 0 948 957 beschreibt die Verwendung von Copolymerisaten monoethylenisch ungesättigter Carbonsäuren als Solubilisatoren. R. Haag beschreibt in Angew. Chemie 2004, 116, S. 280 - 284 supramolekulare Wirkstoff-Transportsysteme auf der Basis polymerer Kern-Schale-Architekturen. Dabei sind auch dendritische Kern-Schale-Architekturen, basierend auf hochverzweigtem Polyglycerin und Poly(ethylenimin) beschrieben.

Die unveröffentlichte deutsche Patentanmeldung 10 2004 037 850.9 beschreibt wässrige Zubereitungen von nicht oder nur gering wasserlöslichen Wirkstoffen, die erhältlich sind, indem man in Gegenwart einer wässrigen Suspension der Wirkstoffpartikel eine erste Emulsionspolymerisation unter Erhalt einer wässrigen Dispersion von Polymer-Wirkstoff-Partikeln durchführt und diese anschließend einer zweiten Emulsionspolymerisation in Gegenwart wenigstens eines neutralen monoethylenisch ungesättigten Monomeren unterzieht.

Der Einsatz von hyperverzweigten Polymeren zur Herstellung von wässrigen Wirkstoffzusammensetzungen von in Wasser schwer löslichen Wirkstoffen ist in den zuvor genannten Dokumenten nicht beschrieben. Es ist jedoch bekannt, solche Polymere für eine Vielzahl verschiedener Verwendungszwecke einzusetzen. So beschreibt beispielsweise die WO 2004/037881 Substrate, die auf ihrer Oberflächen wenigstens ein hyperverzweigtes Polymer enthalten, das Urethan- und/oder Harnstoffgruppen aufweist. Die WO 2004/094505 beschreibt Stabilisatoren, welche Kunststoffe gegen Schädigung durch Wärme, UV-Strahlung, etc. schützen, die über eine Ankergruppe mit einem hochverzweigten Polymer kovalent verbunden sind.

Der vorliegenden Erfindung liegt daher die Aufgabe zu Grunde, Zubereitungen von in Wasser unlöslichen bzw. schwerlöslichen Wirkstoffen, insbesondere von Wirkstoffen für die Pharmazie, die Kosmetik, den Pflanzenschutz oder für den Materialschutz bereitzustellen. Diese Wirkstoffzusammensetzungen sollten leicht herstellbar sein und keinen oder nur einen sehr geringen Gehalt an flüchtigen organischen Substanzen aufweisen. Weiterhin ist eine hohe Stabilität der resultierenden wässrigen Wirkstoffzusammensetzungen im Hinblick auf Entmischungsvorgänge bei langer Lagerung und beim Verdünnen mit Wasser wünschenswert.

Überraschenderweise wurde gefunden, dass die Aufgabe durch den Einsatz hyperverzweigter stickstoffhaltiger Polymere als Solubilisatoren gelöst werden kann.

Gegenstand der Erfindung ist daher eine Wirk- oder Effektstoffzusammensetzung, die
A) wenigstens ein stickstoffatomhaltiges hyperverzweigtes Polymer, und
B) wenigstens einen Wirk- oder Effektstoff, der in Wasser bei 25 °C und 1013 mbar eine Löslichkeit unterhalb 10 g/l aufweist,
enthält.

Die erfindungsgemäß eingesetzten hyperverzweigten Polymere sind in vorteilhafter Weise geeignet, wasserunlösliche (bzw. nur gering wasserlösliche) Wirk- und Effektstoffe in wässriger Phase zu stabilisieren und ermöglichen daher die Herstellung wässriger Formulierungen derartiger Wirk- und Effektstoffe. Sie eignen sich auch zur Herstellung fester Formulierungen dieser Wirk- und Effektstoffe, die sich in eine wässrige Formulierung, z. B. als Handels-, Darreichungs- oder Wirkform, überführen lassen. Dies kann auch erst nach Applikation der feste Zusammensetzung erfolgen (z. B. im Verdauungstrakt eines Organismus, etc.).

Die mit den erfindungsgemäß eingesetzten Polymeren erzielte "Löslichkeitsverbesserung" wird im Rahmen der vorliegenden Erfindung daher breit verstanden. Dazu zählt zum einen die Stabilisierung heterogener Systeme, bei denen der Wirkstoff als emulgierte bzw. dispergierte Phase (disperse Phase) in einem wässrigen Medium als kontinuierliche Phase vorliegt. Dazu zählt weiterhin die Stabilisierung von Übergangsstufen zu homogenen Lösungen, wie kolloidale Lösungen, etc., bis hin zu molekulardispersen Lösungen. Dazu zählt auch eine Löslichkeitsverbesserung im Sinne einer Solubilisierung, bei der die schlecht wasserlöslichen oder wasserunlöslichen Stoffe in klare, höchstens opaleszierende wässrige Lösungen überführt werden. Dazu zählt schließlich auch die Befähigung zur Bildung so genannter "fester Lösungen".

Eine geringe (schlechte) Löslichkeit bedeutet im Rahmen dieser Erfindung eine Löslichkeit des Wirk- bzw. Effektstoffs in Wasser von unterhalb 10 g/l, insbesondere unterhalb 1 g/l und speziell unterhalb 0,1 g/l bei 25 °C und 1013 mbar.

Die unter Verwendung der stickstoffatomhaltigen hyperverzweigten Polymere hergestellten wässrigen Wirkstoffzusammensetzungen von in Wasser unlöslichen Wirk- bzw. Effektstoffen umfassen neben einem wässrigen Medium als kontinuierliche Phase wenigstens einen in der kontinuierlichen Phase solubilisierten oder dispergierten Wirkstoff und/oder Effektstoff, der in Wasser bei 25 °C/1013 mbar eine Löslichkeit unterhalb 10 g/l, insbesondere unterhalb 1 g/l und speziell unterhalb 0,1 g/l aufweisen, sowie wenigstens ein stickstoffatomhaltiges hyperverzweigtes Polymer.

Der Wirkstoff liegt in der kontinuierlichen wässrigen Phase in äußerst feinteiliger Form vor. Dies kann beispielsweise darauf zurückzuführen sein, dass der Wirkstoff in der wässrigen Phase mit den Polymeren A) Aggregate bildet. Diese Aggregate weisen in der Regel mittlere Teilchengrößen unterhalb 1 µm, häufig unterhalb 500 nm, insbesondere unterhalb 400 nm, speziell unterhalb 300 nm auf. Je nach Art des Polymeren und des Wirkstoffs bzw. Effektstoffs sowie abhängig von den Konzentrationsverhältnissen können die Aggregate auch so klein werden, dass sie nicht mehr in Form nachweisbarer, diskreter Partikel sondern in gelöster Form vorliegen (Teilchengröße < 10 nm).

Die hier angegebenen Teilchengrößen sind gewichtsmittlere Teilchengrößen, wie sie durch dynamische Lichtstreuung ermittelt werden können. Verfahren hierzu sind dem Fachmann geläufig, beispielsweise aus H. Wiese in D. Distler, Wässrige Polymerdispersionen, Wiley-VCH 1999, Kapitel 4.2.1, S. 40ff und dort zitierte Literatur sowie H. Auweter, D. Horn, J. Colloid Interf. Sci. 105 (1985) 399, D. Lilge, D. Horn, Colloid Polym. Sci. 269 (1991) 704 oder H. Wiese, D. Horn, J.Chem. Phys. 94 (1991) 6429.

Die Begriffe "wässriges Medium" und "wässrige Phase" umfassen hier und im Folgenden Wasser, wässrige Mischungen von Wasser mit bis zu 10 Gew.-%, bezogen auf die Mischung, an organischen Lösungsmitteln, die mit Wasser mischbar sind, und Lösungen von Feststoffen in Wasser oder in den wässrigen Mischungen. Beispiele für mit Wasser mischbare Lösungsmittel umfassen C₃-C₄-Ketone wie Aceton und Methylethylketon, cyclische Ether wie Dioxan und Tetrahydrofuran, C₁-C₄-Alkanole wie Methanol, Ethanol, n-Propanol, Isopropanol, n-Butanol, tert.-Butanol, Polyole und deren Mono-und Dimethylether wie Glykol, Propandiol, Ethylenglykolmonomethylether, Diethylenglykol, Diethylenglykolmonomethylether, Diethylenglykoldimethylether, Glycerin, weiterhin C₂-C₃-Nitrile wie Acetonitril und Propionitril, Dimethylsulfoxyd, Dimethylformamid, Formamid, Acetamid, Dimethylacetamid, Butyrolacton, 2-Pyrrolidon und N-Methylpyrrolidon.

Der Begriff "Funktionalität" steht hier und im Folgenden für die mittlere Anzahl der jeweiligen funktionellen Gruppen pro Molekül bzw. pro Polymerkette.

Ein Vorteil der erfindungsgemäßen Wirkstoffzusammensetzungen ist, dass sie auch lösungsmittelarm (Gehalt an flüchtigen Lösungsmitteln < 10 Gew.-%, bezogen auf das Gewicht der Wirkstoffzusammensetzung) oder sogar lösungsmittelfrei (Gehalt an flüchtigen Lösungsmitteln < 1 Gew.-%, bezogen auf das Gewicht der Wirkstoffzusammensetzung) formuliert werden können.

Ein weiterer Vorteil ist darin zu sehen, dass sich die erfindungsgemäßen wässrigen Wirkstoffzusammensetzungen in der Regel zu einem redispergierbaren Pulver trocknen lassen. D.h. durch Entfernen der wässrigen Phase während des Trocknens erhält man ein feinteiliges Pulver, das sich ohne weiteres in Wasser lösen oder dispergieren lässt, ohne dass eine nennenswerte Teilchenvergrößerung eintritt.

Im Rahmen der vorliegenden Erfindung umfasst der Begriff "hyperverzweigte Polymere" ganz allgemein Polymere, die sich durch eine verzweigte Struktur und eine hohe Funktionalität auszeichnen. Zur allgemeinen Definition hyperverzweigter Polymere wird auch auf P. J. Flory, J. Am. Chem. Soc. 1952, 74, 2718 und H. Frey et al., Chem. Eur. J. 2000, 6, Nr. 14, 2499 Bezug genommen. Zu den "hyperverzweigten Polymeren" im Sinne der Erfindung zählen Sternpolymere, Dendrimere und davon verschiedene hochmolekulare Polymere, wie z. B. Kammpolymere. "Sternpolymere" sind Polymere, bei denen von einem Zentrum drei oder mehr Ketten ausgehen. Das Zentrum kann dabei ein einzelnes Atom oder eine Atomgruppe sein. "Dendrimere" (hyperverzweigte Polymere, Kaskadenpolymere, Arborole (= Dendrimere mit Hydroxylgruppen), isotrop verzweigte Polymere, isoverzweigte Polymere, Starburst-Polymere) sind molekular einheitliche Makromoleküle mit einem hochsymmetrischen Aufbau. Dendrimere leiten sich strukturell ab von den Sternpolymeren, wobei die einzelnen Ketten jeweils ihrerseits sternförmig verzweigt sind. Sie entstehen ausgehend von kleinen Molekülen durch eine sich ständig wiederholende Reaktionsfolge, wobei immer höhere Verzweigungen resultieren, an deren Enden sich jeweils funktionelle Gruppen befinden, die wiederum Ausgangspunkt für weitere Verzweigungen sind. So wächst mit jedem Reaktionsschritt die Zahl der Monomer-Endgruppen exponentiell an, wobei am Ende eine kugelförmige Baumstruktur entsteht. Ein charakteristisches Merkmal der Dendrimere ist die Anzahl der zu ihrem Aufbau durchgeführten Reaktionsstufen (Generationen). Auf Grund ihres einheitlichen Aufbaus weisen Dendrimere in der Regel eine definierte Molmasse auf.

Bevorzugt geeignet sind sowohl molekular wie strukturell uneinheitliche hyperverzweigte Polymere, die Seitenketten unterschiedlicher Länge und Verzweigung sowie eine Molmassenverteilung aufweisen.

Zur Synthese dieser hyperverzweigten Polymere eignen sich insbesondere so genannte ABₓ-Monomere. Diese weisen zwei verschiedene funktionelle Gruppen A und B auf, die unter Bildung einer Verknüpfung miteinander reagieren können. Die funktionelle Gruppe A ist dabei nur einmal pro Molekül enthalten und die funktionelle Gruppe B zweifach oder mehrfach. Durch die Reaktion der besagten ABₓ-Monomere miteinander entstehen im Wesentlichen unvernetzte Polymere mit regelmäßig angeordneten Verzweigungsstellen. Die Polymere weisen an den Kettenenden fast ausschließlich B-Gruppen auf. Nähere Einzelheiten sind beispielsweise in Journal of Molecular Science, Rev. Macromol. Chem. Phys., C37(3), 555-579 (1997) zu finden.

Die erfindungsgemäß eingesetzten hyperverzweigten Polymere weisen vorzugsweise einen Verzweigungsgrad (Degree of Branching, DB) entsprechend einer mittleren Anzahl von dendritischen Verknüpfungen und terminalen Einheiten pro Molekül von 10 bis 100 %, bevorzugt 10 bis 90 % und insbesondere 10 bis 80 %, auf. Zur Definition des "Degree of Branching" wird auf H. Frey et al., Acta Polym. 1997, 48, 30 verwiesen.

Hyperverzweigte Polymere, d. h. molekular und strukturell uneinheitliche Polymere, werden bevorzugt eingesetzt. Diese sind in der Regel einfacher und somit wirtschaftlicher herstellbar als Dendrimere. Zur Erzielung einer vorteilhaften Oberflächenmodifizierung können aber selbstverständlich auch strukturell und molekular einheitliche dendrimere Polymere und Sternpolymere eingesetzt werden.

Vorzugsweise sind die stickstoffatomhaltigen hyperverzweigten Polymere A) ausgewählt unter Polyurethanen, Polyharnstoffen, Polyamiden, Polyesteramiden, Polyesteraminen und Mischungen davon.

Vorzugsweise weisen die erfindungsgemäß eingesetzten hyperverzweigten Polymere neben den bei der Synthese der hyperverzweigten Struktur resultierenden Gruppen (z. B. bei hyperverzweigten Polyurethanen Urethan- und/oder Harnstoffgruppen bzw. weitere aus der Reaktion von Isocyanatgruppen hervorgehende Gruppen; bei hyperverzweigten Polyamiden Amidgruppen, etc.) wenigstens vier weitere funktionelle Gruppen auf. Die maximale Anzahl dieser funktionellen Gruppen ist in der Regel nicht kritisch. Sie beträgt jedoch vielfach nicht mehr als 100. Bevorzugt beträgt der Anteil an funktionellen Gruppen 4 bis 100, speziell 5 bis 30, und spezieller 6 bis 20.

Bevorzugt sind Polymere, die ein gewichtsmittleres Molekulargewicht im Bereich von etwa 500 bis 100000, bevorzugt 750 bis 50000, insbesondere 1000 bis 30000 aufweisen.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc. Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Der Ausdruck "Alkylen" im Sinne der vorliegenden Erfindung steht für geradkettige oder verzweigte Alkandiyl-Gruppen mit 1 bis 4 Kohlenstoffatomen, z. B. Methylen, 1,2-Ethylen, 1,3-Propylen, etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

### I) Hyperverzweigte Polyurethane

In einer ersten Ausführung umfasst die erfindungsgemäße wässrige Wirkstoffzusammensetzung wenigstens ein hyperverzweigtes Polyurethan-Polymer.

Der Begriff "Polyurethane" umfasst im Rahmen dieser Erfindung nicht nur solche Polymere, deren Wiederholungseinheiten durch Urethangruppen miteinander verbunden sind, sondern ganz allgemein Polymere, die durch Umsetzung wenigstens eines Di-und/oder Polyisocyanats mit wenigstens einer Verbindung erhältlich sind, die mindestens eine gegenüber Isocyanatgruppen reaktive Gruppe aufweist. Dazu zählen Polymere, deren Wiederholungseinheiten neben Urethangruppen auch durch Harnstoff-, Allophanat-, Biuret-, Carbodiimid-, Amid-, Uretonimin-, Uretdion-, Isocyanurat- oder Oxazolidon-(Oxazolidinon-)Gruppen verbunden sind (siehe beispielsweise Kunststofftaschenbuch, Saechtling, 26. Aufl., S. 491ff, Carl-Hanser-Verlag, München 1995). Der Begriff "Polyurethane" umfasst insbesondere Polymere, die Urethan- und/oder Harnstoffgruppen aufweisen.

Vorzugsweise weisen die erfindungsgemäß eingesetzten hyperverzweigten Polymere neben Urethan- und/oder Harnstoffgruppen (bzw. weiteren aus der Reaktion von Isocyanatgruppen hervorgehenden Gruppen) wenigstens vier weitere funktionelle Gruppen auf. Bevorzugt beträgt der Anteil an funktionellen Gruppen 4 bis 100, besonders bevorzugt 5 bis 30 und insbesondere 6 bis 20.

Bevorzugt sind Polyurethane, die ein gewichtsmittleres Molekulargewicht im Bereich von etwa 500 bis 100000, bevorzugt 1000 bis 50000, aufweisen.

Bevorzugt liegt ihr Gehalt an Urethan- und/oder Harnstoffgruppen (und, falls vorhanden, weiteren durch Umsetzung einer Isocyanatgruppe mit einer demgegenüber reaktiven Gruppe mit aktivem Wasserstoffatom erhaltenen Gruppen) in einem Bereich von 0,5 bis 10 mol/kg, besonders bevorzugt 1 bis 10 mol/kg, insbesondere 2 bis 8 mol/kg. Die Synthese von erfindungsgemäß einsetzbaren hyperverzweigten Polyurethanen und Polyharnstoffen kann beispielsweise wie im Folgenden geschildert erfolgen.

Zur Synthese der hyperverzweigten Polyurethane und Polyharnstoffe werden bevorzugt ABₓ-Monomere eingesetzt, die sowohl Isocyanat-Gruppen sowie Gruppen, die mit Isocyanat-Gruppen unter Bildung einer Verknüpfung reagieren können, aufweisen. Bei x handelt es sich um eine natürliche Zahl zwischen 2 und 8. Bevorzugt beträgt x 2 oder 3. Entweder handelt es sich bei A um die Isocyanat-Gruppen und bei B um mit diesen reaktive Gruppen oder es kann der umgekehrte Fall vorliegen.

Bei den mit den Isocyanat-Gruppen reaktiven Gruppen handelt es sich bevorzugt um OH-, NH₂-, NRH-, SH- oder COOH-Gruppen.

Die ABₓ-Monomere sind in bekannter Art und Weise mittels verschiedener Techniken herstellbar.

ABₓ-Monomere können beispielsweise nach der von WO 97/02304 offenbarten Methode unter Anwendung von Schutzgruppentechniken synthetisiert werden. Beispielhaft sei diese Technik an der Herstellung eines AB₂-Monomers aus 2,4-Toluylendiisocyanat (TDI) und Trimethylolpropan erläutert. Zunächst wird eine der Isocyanat-Gruppen des TDI in bekannter Art und Weise verkappt, beispielsweise durch Umsetzung mit einem Oxim. Die verbleibende freie NCO-Gruppe wird mit Trimethylolpropan umgesetzt, wobei eine der drei OH-Gruppen mit der Isocyanat-Gruppe reagiert. Nach Abspalten der Schutzgruppe wird ein Molekül mit einer Isocyanat-Gruppe und 2 OH-Gruppen erhalten.

Besonders vorteilhaft können die ABₓ-Moleküle nach der von DE-A 199 04 444 offenbarten Methode synthetisiert werden, bei der keine Schutzgruppen erforderlich sind. Bei dieser Methode werden Di- oder Polyisocyanate eingesetzt und mit Verbindungen, die mindestens zwei mit Isocyanatgruppen reaktive Gruppen aufweisen, umgesetzt. Zumindest einer der Reaktionspartner weist Gruppen mit gegenüber dem anderen Reaktionspartner unterschiedlicher Reaktivität auf. Bevorzugt weisen beide Reaktionspartner Gruppen mit gegenüber dem anderen Reaktionspartner unterschiedlicher Reaktivität auf. Die Reaktionsbedingungen werden so gewählt, dass nur bestimmte reaktive Gruppen miteinander reagieren können.

Weiterhin können ABₓ-Moleküle wie in der deutschen Patentanmeldung P 102 04 979.3 beschrieben hergestellt werden. Hier werden durch Verkappungsmittel geschützte Isocyanatgruppen mit Polyaminen zu Polyharnstoffen umgesetzt.

Als Di- oder Polyisocyanate kommen die nach dem Stand der Technik bekannten und nachfolgend beispielhaft genannten aliphatischen, cycloaliphatischen, araliphatischen und aromatischen Di- oder Polyisocyanate in Frage. Zu nennen sind hier vorzugsweise 4,4'-Diphenylmethandiisocyanat, die Mischungen aus monomeren Diphenylmethandiisocyanaten und oligomeren Diphenylmethandiisocyanaten (Polymer-MDI), Tetramethylendiisocyanat, Tetramethylendiisocyanat-Trimere, Hexamethylendiisocyanat, Hexamethylendiisocyanat-Trimere, Isophorondiisocyanat-Trimer, 4,4'-Methylenbis(cyclohexyl)-diisocyanat, Xylylendiisocyanat, Tetramethylxylylendiisocyanat, Dodecyldiisocyanat, Lysinalkylesterdiisocyanat, wobei Alkyl für C₁-C₁₀-steht, 1,4-Diisocyanatocyclohexan oder 4-Isocyanatomethyl-1,8-octamethylendiisocyanat.

Besonders bevorzugt zum Aufbau der Polyurethane und Polyharnstoffe sind Di- oder Polyisocyanate geeignet, die NCO-Gruppen unterschiedlicher Reaktivität aufweisen. Genannt seien hier 2,4-Toluylendiisocyanat (2,4-TDI), 2,4'-Diphenylmethandiisocyanat (2,4'-MDI), Triisocyanatotoluol, Isophorondiisocyanat (IPDI), 2-Butyl-2-ethylpentamethylendiisocyanat, 2,2,4- oder 2,4,4-Trimethyl-1,6-hexamethylendiisocyanat, 2-Isocyanatopropylcyclohexylisocyanat, 3(4)-Isocyanatomethyl-1-methylcyclohexylisocyanat, 1,4-Diisocyanato-4-methylpentan, 2,4'-Methylenbis(cyclohexyl)diisocyanat und 4-Methylcyclohexan-1,3-diisocyanat (H-TDI).

Weiterhin sind zum Aufbau der Polyurethane und Polyharnstoffe Isocyanate geeignet, deren NCO-Gruppen zunächst gleich reaktiv sind, bei denen sich jedoch durch Erstaddition eines Reaktanden an einer NCO-Gruppe ein Reaktivitätsabfall bei der zweiten NCO-Gruppe induzieren lässt. Beispiele dafür sind Isocyanate, deren NCO-Gruppen über ein delokalisiertes p-Elektronensystem gekoppelt sind, z. B. 1,3- und 1,4-Phenylendiisocyanat, 1,5-Naphthylendiisocyanat, Diphenyldiisocyanat, Tolidindiisocyanat oder 2,6-Toluylendiisocyanat.

Weiterhin können beispielsweise Oligo- oder Polyisocyanate verwendet werden, die sich aus den oben genannten Di- oder Polyisocyanaten oder deren Mischungen durch Verknüpfung mittels Urethan-, Allophanat-, Harnstoff-, Biuret-, Uretdion-, Amid-, Isocyanurat-, Carbodiimid-, Uretonimin-, Oxadiazintrion- oder Iminooxadiazindion-Strukturen herstellen lassen.

Als Verbindungen mit mindestens zwei mit Isocyanaten reaktiven Gruppen werden vorzugsweise di-, tri- oder tetrafunktionelle Verbindungen eingesetzt, deren funktionelle Gruppen gegenüber NCO-Gruppen eine unterschiedliche Reaktivität aufweisen. Bevorzugt zur Herstellung von Polyurethanen und Polyharnstoff-Polyurethanen sind Verbindungen mit mindestens einer primären und mindestens einer sekundären Hydroxylgruppe, mindestens einer Hydroxylgruppe und mindestens einer Mercaptogruppe, besonders bevorzugt mit mindestens einer Hydroxylgruppe und mindestens einer Aminogruppe im Molekül, insbesondere Aminoalkohole, Aminodiole und Aminotriole, da die Reaktivität der Aminogruppe gegenüber der Hydroxylgruppe bei der Umsetzung mit Isocyanat deutlich höher ist.

Beispiele für die genannten Verbindungen mit mindestens zwei mit Isocyanaten reaktiven Gruppen sind Propylenglykol, Glycerin, Mercaptoethanol, Ethanolamin, N-Methylethanolamin, Diethanolamin, Ethanolpropanolamin, Dipropanolamin, Diisopropanolamin, 2-Amino-1,3-propandiol, 2-Amino-2-methyl-1,3-propandiol oder Tris(hydroxymethyl)aminomethan. Weiterhin sind auch Mischungen der genannten Verbindungen einsetzbar.

Für die Herstellung von Polyharnstoffen werden vorzugsweise Isocyanat-reaktive Produkte eingesetzt, die mindestens zwei Aminogruppen im Molekül aufweisen.

Dies sind zum Beispiel Ethylendiamin, N-Alkylethylendiamin, Propylendiamin, N-Alkylpropylendiamin, Hexamethylendiamin, N-Alkylhexamethylendiamin, Diaminodicyclohexylmethan, Phenylendiamin, Isophorondiamin, Amin-terminierte Polyoxyalkylenpolyole (so genannte Jeffamine), Bis(aminoethyl)amin, Bis(aminopropyl)amin, Bis(aminohexyl)amin, Tris(aminoethyl)amin, Tris(aminopropyl)amin, Tris(aminohexyl)amin, Trisaminohexan, 4-Aminomethyl-1,8-octamethylendiamin, N'-(3-Aminopropyl)-N,N-dimethyl-1,3-propandiamin, Trisaminononan oder Melamin. Weiterhin sind auch Mischungen der genannten Verbindungen einsetzbar.

Die Herstellung eines ABₓ-Moleküls zur Herstellung eines Polyurethans aus einem Diisocyanat und einem Aminodiol sei hier beispielhaft erläutert. Hierbei wird zunächst ein Mol eines Diisocyanats mit einem Mol eines Aminodiols bei niedrigen Temperaturen, vorzugsweise im Bereich zwischen -10 bis 30 °C, umgesetzt. In diesem Temperaturbereich erfolgt eine praktisch vollständige Unterdrückung der Urethanbildungsreaktion und die NCO-Gruppen des Isocyanats reagieren ausschließlich mit der Aminogruppe des Aminodiols. Das gebildete ABₓ-Molekül, hier ein AB₂-Typ, weist eine freie NCO-Gruppe sowie zwei freie OH-Gruppen auf und kann zur Synthese eines hyperverzweigten Polyurethans eingesetzt werden.

Durch Erwärmung und/oder Katalysatorzugabe kann dieses AB₂-Molekül intermolekular zu einem hyperverzweigten Polyurethan reagieren. Die Synthese des hyperverzweigten Polyurethans kann vorteilhaft ohne vorherige Isolierung des AB₂-Moleküls in einem weiteren Reaktionsschritt bei erhöhter Temperatur erfolgen, vorzugsweise im Bereich zwischen 30 und 80 °C. Bei Verwendung des geschilderten AB₂-Moleküls mit zwei OH-Gruppen und einer NCO-Gruppe entsteht ein hyperverzweigtes Polymer, welches pro Molekül eine freie NCO-Gruppe sowie - je nach Polymerisationsgrad - eine mehr oder weniger große Zahl von OH-Gruppen aufweist. Die Reaktion kann bis zu hohen Umsätzen durchgeführt werden, wodurch sehr hochmolekulare Strukturen erhalten werden. Sie kann aber auch beispielsweise durch Zugabe geeigneter monofunktioneller Verbindungen oder durch Zugabe einer der Ausgangsverbindungen zur Herstellung des AB₂-Moleküls beim Erreichen des gewünschten Molekulargewichts abgebrochen werden. Je nach der zum Abbruch verwendeten Ausgangsverbindung entstehen entweder vollständig NCO-terminierte oder vollständig OH-terminierte Moleküle.

Alternativ kann beispielsweise auch ein AB₂-Molekül aus 1 Mol Glycerin und 2 Mol 2,4-TDI hergestellt werden. Bei tiefer Temperatur reagieren vorzugsweise die primären Alkoholgruppen sowie die Isocyanat-Gruppe in 4-Stellung, und es wird ein Addukt gebildet, welches eine OH-Gruppe und zwei Isocyanat-Gruppen aufweist, und das wie geschildert bei höheren Temperaturen zu einem hyperverzweigten Polyurethan umgesetzt werden kann. Es entsteht zunächst ein hyperverzweigtes Polymer, welches eine freie OH-Gruppe sowie - je nach Polymerisationsgrad - eine mehr oder weniger große Zahl von NCO-Gruppen aufweist.

Die Herstellung der hyperverzweigten Polyurethane und Polyharnstoffe kann prinzipiell ohne Lösungsmittel, bevorzugt aber in Lösung erfolgen. Als Lösungsmittel prinzipiell geeignet sind alle bei der Umsetzungstemperatur flüssigen und gegenüber den Monomeren und Polymeren inerten Verbindungen.

Andere Produkte sind durch weitere Synthesevarianten zugänglich. Beispielhaft seien an dieser Stelle genannt:
AB₃-Moleküle lassen sich beispielsweise durch Reaktion von Diisocyanaten mit Verbindungen mit mindestens 4 gegenüber Isocyanaten reaktiven Gruppen erhalten. Beispielhaft sei die Umsetzung von Toluylendiisocyanat mit Tris(hydroxymethyl)aminomethan genannt.

Zum Abbruch der Polymerisation können auch polyfunktionelle Verbindungen eingesetzt werden, die mit den jeweiligen A-Gruppen reagieren können. Auf diese Art und Weise können mehrere kleine hyperverzweigte Moleküle zu einem großen hyperverzweigten Molekül verknüpft werden.

Hyperverzweigte Polyurethane und Polyharnstoffe mit kettenverlängerten Ästen lassen sich beispielsweise erhalten, indem zur Polymerisationsreaktion neben den ABₓ-Molekülen zusätzlich im molaren Verhältnis 1:1 ein Diisocyanat und eine Verbindung, die zwei mit Isocyanatgruppen reaktive Gruppen aufweist, eingesetzt werden. Diese zusätzlichen AA- bzw. BB-Verbindungen können auch noch über weitere funktionelle Gruppen verfügen, die bei den Reaktionsbedingungen aber nicht reaktiv gegenüber den A- oder B-Gruppen sein dürfen. Auf diese Art und Weise können weitere Funktionalitäten in das hyperverzweigte Polymer eingebracht werden.

Weitere geeignete Synthesevarianten für hyperverzweigte Polymere finden sich in der DE-A-100 13 187, DE-A-100 30 869 sowie den deutschen Patentanmeldungen P 103 51 401.5 und P 10 2004 006304.4

### II) Hyperverzweigte Polyamide

Hyperverzweigte Polyamide sind beispielsweise in der US 4,507,466, US 6,541,600, US-A-2003055209, US 6,300,424, US 5,514,764 und WO 92/08749 beschrieben, worauf hier in vollem Umfang Bezug genommen wird.

Eine geeignete Vorgehensweise zur Herstellung hyperverzweigter Polyamide geht aus von polyfunktionellen Aminen und Polycarbonsäuren, wobei zumindest eine polyfunktionelle Verbindung eingesetzt wird, die drei oder mehr als drei (z. B. 4, 5, 6, etc.) funktionelle Gruppen aufweist. Formal werden bei dieser Vorgehensweise dann eine erste Klasse von Monomeren mit zwei gleichen funktionellen Gruppen A₂ (z. B eine Dicarbonsäure oder ein Diamin) mit einer zweiten Klasse von Monomeren Bₙ umgesetzt, wobei diese zweite Klasse, wenigstens eine Verbindung mit mehr als gleichen funktionellen Gruppen (z. B wenigstens eine Tricarbonsäure (n =3) oder eine höher als dreiwertige Carbonsäure bzw. wenigstens ein Triamin (n = 3) oder ein höher als dreiwertiges Amin) umfasst. Vorzugsweise umfasst die zweite Klasse von Monomeren wenigstens ein zweiwertiges Monomer B₂, welches zwei zu den Monomeren A₂ komplementäre funktionelle Gruppen aufweist. Vorzugsweise weisen die Monomere Bₙ eine mittlere Funktionalität von wenigstens 2,1 (n = 2,1) auf. Bevorzugt werden zur Herstellung von hyperverzweigten Polyamiden nach dieser Variante die Monomere A₂ in einem molaren Überschuss gegenüber den Monomeren Bₙ eingesetzt. Vorzugsweise liegt das molare Verhältnis von Monomere A₂ zu Monomeren Bₙ in einem Bereich von 1:1 bis 20:1, besonders bevorzugt von 1,1:1 bis 10:1, insbesondere 1,2:1 bis 5:1. In einer bevorzugten Ausführungsform wird zunächst ein hyperverzweigtes Prepolymer mit endständigen Gruppen A hergestellt und anschließend mit wenigstens einem Monomer B₂ und/oder Bₙ weiter umgesetzt. Zur Herstellung des Prepolymers werden vorzugsweise Monomere A₂ und Monomere Bₙ in einem molaren Verhältnis von 1:1 bis 20:1, besonders bevorzugt von 1,1:1 bis 10:1, insbesondere 1,2:1 bis 5:1, eingesetzt.

Eine weitere geeignete Vorgehensweise zur Herstellung hyperverzweigter Polyamide geht aus von polyfunktionellen Aminocarbonsäuren, wobei zumindest eine polyfunktionelle Verbindung eingesetzt wird, die drei oder mehr als drei (z. B. 4, 5, 6, etc.) funktionelle Gruppen aufweist, d.h. ein so genanntes ABₓ-Monomer (x ist größer als oder gleich 2). Diese können dann mit weiteren Monomeren AB, A₂ und/oder B₂ umgesetzt werden.

Geeignete Dicarbonsäuren sind beispielsweise Oxalsäure, Malonsäure, Bernsteinsäure, Glutarsäure, Adipinsäure, Pimelinsäure, Korksäure, Azelainsäure, Sebacinsäure,Undecan-α,ω-dicarbonsäure,Dodecan-α,ω-dicarbon-säure, cis- und trans-Cyclohexan-1, 2-dicarbonsäure, cis- und trans-Cyclohexan-1, 3-dicarbonsäure, cis-und trans-Cyclohexan-1, 4-dicarbonsäure, cis- und trans-Cyclopentan-1,2-dicarbonsäure, cis- und trans-Cyclopentan-1, 3-dicarbonsäure, Phthalsäure, Isophthalsäure, Terephthalsäure und Mischungen davon.

Die oben genannten Dicarbonsäuren können auch substituiert sein. Geeignete substituierte Dicarbonsäuren können einen oder mehrere Reste aufweisen, die vorzugsweise ausgewählt sind unter Alkyl, Cycloalkyl und Aryl, wie eingangs definiert. Geeignete substituierte Dicarbonsäuren sind beispielsweise 2-Methylmalonsäure, 2-Ethylmalonsäure, 2-Phenylmalonsäure, 2-Methylbernsteinsäure, 2-Ethylbernsteinsäure, 2-Phenylbernsteinsäure, Itaconsäure, 3,3-Dimethylglutarsäure, etc.

Die Dicarbonsäuren lassen sich entweder als solche oder in Form von Derivaten einsetzen. Geeignete Derivate sind Anhydride und deren Oligomere und Polymere, Mono-und Diester, bevorzugt Mono- und Dialkylester, und Säurehalogenide, vorzugsweise Chloride. Geeignete Ester sind Mono- oder Dimethylester, Mono- oder Diethylester, sowie Mono- und Diester von höheren Alkoholen wie beispielsweise n-Propanol, Isopropanol, n-Butanol, Isobutanol, tert. -Butanol, n-Pentanol, n-Hexanol, etc, ferner Mono- und Divinylester sowie gemischte Ester, bevorzugt Methylethylester.

Im Rahmen der vorliegenden Erfindung ist es auch möglich, ein Gemisch aus einer Dicarbonsäure und einem oder mehreren ihrer Derivate einzusetzen. Gleichfalls ist es im Rahmen der vorliegenden Erfindung möglich, ein Gemisch mehrerer verschiedener Derivaten von einer oder mehreren Dicarbonsäuren einzusetzen.

Besonders bevorzugt setzt man Bernsteinsäure, Glutarsäure, Adipinsäure, Phthalsäure, Isophthalsäure, Terephthalsäure oder deren Mono- oder Dimethylester ein. Ganz besonders bevorzugt setzt man Adipinsäure ein.

Geeignete polyfunktionelle Amine zur Herstellung hyperverzweigter Polyamide weisen 2 oder mehr als 2 (z. B. 3, 4, 5, 6 etc.) zur Amidbildung befähigte primäre oder sekundäre Aminogruppen auf.

Geeignete Diamine sind geradkettige und verzweigte, aliphatische und cycloaliphatische Amine mit im Allgemeinen etwa 2 bis 30, bevorzugt etwa 2 bis 20 Kohlenstoffatomen. Geeignet sind beispielsweise Diamine der allgemeinen Formel R¹-NH-R²-NH-R³, worin R¹ und R³ unabhängig voneinander für Wasserstoff, Alkyl, Cycloalkyl oder Aryl stehen und R² für Alkylen, Cycloalkylen oder Arylen steht. Dazu zählen Ethylendiamin, 1,2-Diaminopropan, 1,3-Diaminopropan, 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,11-Diaminoundecan, 1,12-Diaminododecan, N-Alkylethylendiamine, wie N-Methylethylendiamin und N-Ethylethylendiamin, N,N'-Dialkylethylendiamine, wie N,N'-Dimethylethylendiamin, N-Alkylhexamethylendiamine, wie N-Methylhexamethylendiamin, Piperazin, Bis-(4-aminocyclohexyl)methan, Phenylendiamin, Isophorondiamin, Bis-(2-aminoethyl)ether, 1,2-Bis-(2-aminoethoxy)ethan und Amin-terminierte Polyoxyalkylenpolyole (so genannte Jeffamine oder α,ω-Diaminopolyether), die z. B. durch Aminierung von Polyalkylenoxiden mit Ammoniak herstellbar sind.

Geeignete Triamine sind z. B. Bis-(2-aminoethyl)amin (= Diethylentriamin), N,N'-Diethyldiethylentriamin, Bis-(3-aminopropyl)amin, Bis-(6-aminohexyl)amin, 4-Aminomethyl-1,8-octamethylendiamin, N'-(3-Aminopropyl)-N,N-dimethyl-1,3-propandiamin, Melamin, etc.

Geeignete höherwertige Amine sind N,N'-Bis-(2-aminoethyl)ethylendiamin (= Triethylentetramin), N,N'-Bis-(2-aminoethyl)-1,3-diaminopropan, N,N'-Bis-(3-aminopropyl)-1,4-diaminobutan (= Spermin), N,N'-Bis(2-Aminoethyl)piperazin, N,N'-Bis(3-Aminopropyl)piperazin, Tris-(2-aminoethyl)amin, Tris-(3-aminopropyl)amin, Tris-(6-aminohexyl)amin etc.

Geeignet sind auch polymere Polyamine. Diese weisen im Allgemeinen ein zahlenmittleres Molekulargewicht von etwa 400 bis 10000, bevorzugt etwa 500 bis 8000 auf. Dazu zählen z. B. Polyamin mit endständigen, primären oder sekundären Aminogruppen, Polyalkylenimine, bevorzugt Polyethylenimine und durch Hydrolyse von Poly-N-vinylamiden, wie z. B. Poly-N-vinylacetamid, erhaltene Vinylamine, die zuvor genannten α,ω-Diamine auf der Basis von aminierten Polyalkylenoxiden sowie Copolymerisate, die α,β-ethylenisch ungesättigte Monomere mit entsprechenden funktionellen Gruppen, z. B. Aminomethylacrylat, Aminoethylacrylat, (N-Methyl)aminoethylacrylat, (N-Methyl)aminoethylmethacrylat etc., einpolymerisiert enthalten.

Die zuvor beschriebenen hyperverzweigten Polyamide können im Allgemeinen bereits als solche zur Herstellung wässriger Formulierungen von nicht oder nur gering löslichen Wirk- und Effektstoffen eingesetzt werden. In einer weiteren Ausführung werden die zuvor beschriebenen hyperverzweigten Polyamide zusätzlich noch einer polymeranalogen Umsetzung unterzogen, wie sie im Folgenden beschrieben wird. Dazu eignen sich beispielsweise Monocarbonsäuren, Monoamine, Mono- oder Polyole, sowie Mono- und Polycarbonsäuren, Aminocarbonsäuren, Mono- und Polyamine, Mono- und Polyole mit speziellen funktionellen Gruppen zur Modifizierung der Eigenschaften der hyperverzweigten Polyamide.

Die Herstellung der hyperverzweigten Polyamide kann in Gegenwart eines üblichen Katalysators erfolgen. Dazu zählen z. B. Metalloxide und -carbonate, starke Säuren, Terephthalate, Titanhalogenide, -alkoxide und -carboxylate, etc. Geeignete Katalysatoren sind beispielsweise in US 2,244,192, US 2,669,556, SU 775 106 und US 3,705,881 offenbart. Weitere geeignete Katalysatoren werden im Folgenden bei den Polyesteramiden genannt.

### III) Hyperverzweigte Polyesteramide

Geeignete hyperverzweigte Polyesteramide sind beispielsweise in der WO 99/16810 und der WO 00/56804 beschrieben, worauf hier in vollem Umfang Bezug genommen wird.

Polyesteramide sind ganz allgemein polymere Verbindungen, die Estergruppen und Amidgruppen aufweisen. Zur Herstellung hyperverzweigter Polyesteramide können prinzipiell wenigstens zweiwertige Verbindungen eingesetzt werden, die ausgewählt sind unter Polycarbonsäuren, Hydroxycarbonsäuren, Aminocarbonsäuren, Aminoalkoholen, Polyaminen, Polyolen und Derivaten der zuvor genannten Verbindungen. Dabei gilt zum einen die Maßgabe, dass die Verbindungen so ausgewählt werden, dass die erhaltenen Polymere sowohl Estergruppen als auch Amidgruppen aufweisen. Dabei gilt weiterhin die Maßgabe, dass die Verbindungen so ausgewählt werden, dass zumindest eine polyfunktionelle Verbindung eingesetzt wird, die drei oder mehr als drei (z. B. 4, 5, 6, etc.) funktionelle Gruppen aufweist.

Eine geeignete Vorgehensweise zur Herstellung hyperverzweigter Polyesteramide geht aus von polyfunktionellen Aminoalkoholen und Polycarbonsäuren, wobei zumindest eine polyfunktionelle Verbindung eingesetzt wird, die drei oder mehr als drei (z. B. 4, 5, 6, etc.) funktionelle Gruppen aufweist.

Eine weitere geeignete Vorgehensweise zur Herstellung hyperverzweigter Polyesteramide geht aus von polyfunktionellen Aminen, polyfunktionellen Alkoholen und Polycarbonsäuren, wobei zumindest eine polyfunktionelle Verbindung eingesetzt wird, die drei oder mehr als drei (z. B. 4, 5, 6, etc.) funktionelle Gruppen aufweist.

Geeignete polyfunktionelle Aminoalkohole zur Herstellung hyperverzweigter Polyesteramide weisen zwei oder mehr als zwei (z. B. 3, 4, 5, 6 etc.) funktionelle Gruppen auf, die ausgewählt sind unter Hydroxylgruppen und primären und sekundären Aminogruppen auf. Definitionsgemäß weisen Aminoalkohole dabei immer wenigstens eine Hydroxylgruppe und wenigstens eine primäre oder sekundäre Aminogruppen auf, Geeignete Aminoalkohole sind geradkettige und verzweigte, aliphatische und cycloaliphatische Aminoalkohole mit im Allgemeinen 2 bis 30, bevorzugt 2 bis 20 Kohlenstoffatomen.

Geeignete zweiwertige Aminoalkohole sind z. B. 2-Aminoethanol (= Monoethanolamin), 3-Amino-1-propanol, 2-Amino-1-propanol, 1-Amino-2-propanol, 2-Amino-3-phenylpropanol, 2-Amino-2-methyl-1-propanol, 2-Amino-1-butanol, 4-Amino-1-butanol, 2-Aminoisobutanol, 2-Amino-3-methyl-1-butanol, 2-Amino-3,3-dimethylbutanol, 1-Amino-1-pentanol, 5-Amino-1-pentanol, 2-Amino-1-pentanol, 2-Amino-4-methyl-1-pentanol, 2-Amino-3-methyl-1-pentanol, 2-Aminocyclohexanol, 4-Aminocyclohexanol, 3-(Aminomethyl)-3,5,5-trimethylcyclohexanol, 2-Amino-1,2-diphenylethanol, 2-Amino-1,1-diphenylethanol, 2-Amino-2-phenylethanol, 2-Amino-1-phenylethanol, 2-(4-Aminophenyl)-ethanol, 2-(2-Aminophenyl)-ethanol, 1-(3-Aminophenyl)ethanol, 2-Amino-1-hexanol, 6-Amino-1-hexanol, 6-Amino-2-methyl-2-heptanol, N-Methylisopropanolamin, N-Ethylisopropanolamin, N-Methylethanolamin, 1-Ethylaminobutan-2-ol, 4-Methyl-4-aminopentan-2-ol, 2-(2-Aminoethoxy)-ethanol, N-(2-Hydroxyethyl)-piperazin, 1-Amino-2-indanol, N-(2-Hydroxyethyl)-anilin, Aminozucker, wie D-Glucoseamin, D-Galactoseamin, 4-Amino-4,6-didesoxy-α-D-glucopyranose, und Mischungen davon.

Geeignete drei- und höherwertige Aminoalkohole sind z. B. N-(2-Hydroxyethyl)ethylendiamin, Diethanolamin, Dipropanolamin, Diisopropanolamin, 2-Amino-1,3-propandiol, 3-Amino-1,2-propandiol, etc.

Geeignete Polycarbonsäuren zur Herstellung hyperverzweigter Polyesteramide sind die zuvor zur Herstellung hyperverzweigter Polyamide beschriebenen. Auf die dort genannten geeigneten und bevorzugten Ausführungen wird in vollem Umfang Bezug genommen.

Geeignete polyfunktionelle Amine zur Herstellung hyperverzweigter Polyesteramide sind die zuvor zur Herstellung hyperverzweigter Polyamide beschriebenen. Auf die dort genannten geeigneten und bevorzugten Ausführungen wird in vollem Umfang Bezug genommen.

Geeignete polyfunktionelle Alkohole zur Herstellung hyperverzweigter Polyesteramide weisen zwei oder mehr als zwei (z. B. 3, 4, 5, 6 etc.) Hydroxylgruppen auf. Dabei können die Hydroxylgruppen auch teilweise oder vollständig durch Mercaptogruppen ersetzt sein.

Geeignete Diole sind geradkettige und verzweigte, aliphatische und cycloaliphatische Alkohole mit im Allgemeinen etwa 2 bis 30, bevorzugt etwa 2 bis 20 Kohlenstoffatomen. Dazu zählen 1,2-Ethandiol, 1,2-Propandiol, 1,3-Propandiol, 1,2-Butandiol, 1,3-Butandiol, 1,4-Butandiol, 2,3-Butandiol, 1,2-Pentandiol, 1,3-Pentandiol, 1,4-Pentandiol, 1,5-Pentandiol, 2,3-Pentandiol, 2,4-Pentandiol 1,2-Hexandiol, 1,3-Hexandiol, 1,4-Hexandiol, 1,5-Hexandiol, 1,6-Hexandiol, 2,5-Hexandiol, 1,2-Heptandiol, 1,7-Heptandiol, 1,2-Octandiol, 1,8-Octandiol, 1,2-Nonandiol, 1,9-Nonandiol, 1,2-Decandiol, 1,10-Decandiol, 1,12-Dodecandiol, 2-Methyl-1,3-propandiol, 2-Methyl-2-butyl-1,3-propandiol, 2,2-Dimethyl-1,3-propandiol, 2,2-Dimethyl-1,4-butandiol, Pinacol, 2-Ethyl-2-butyl-1,3-propandiol, Diethylenglykol, Triethylenglykol, Dipropylenglykol, Tripropylenglykol, Polyalkylenglycole, Cyclopentandiole, Cyclohexandiole, etc.

Geeignete Triole sind z. B. Glycerin, Butan-1,2,4-triol, n-Pentan-1,2,5-triol, n-Pentan-1,3,5-triol, n-Hexan-1,2,6-triol, n-Hexan-1,2,5-triol, Trimethylolpropan, Trimethylolbutan. Geeignete Triole sind weiterhin die Triester von Hydroxycarbonsäuren mit dreiwertigen Alkoholen. Vorzugsweise handelt es sich dabei um Triglyceride von Hydroxycarbonsäuren, wie z. B. Milchsäure, Hydroxystearinsäure und Ricinolsäure. Geeignet sind auch natürlich vorkommende Gemische, die Hydroxycarbonsäuretriglyceride enthalten, insbesondere Ricinusöl. Geeignete höherwertige Polyole sind z. B. Zuckeralkohole und deren Derivate, wie Erythrit, Pentaerythrit, Dipenterythrit, Threit, Inosit und Sorbit. Geeignet sind auch Umsetzungsprodukte der Polyole mit Alkylenoxiden, wie Ethylenoxid und/oder Propylenoxid. Es können auch höhermolekulare Polyole mit einem zahlenmittleren Molekulargewicht im Bereich von etwa 400 bis 6000 g/mol, bevorzugt 500 bis 4000 g/mol, eingesetzt werden. Dazu zählen z. B. Polyesterole auf Basis von aliphatischen, cycloaliphatischen und/oder aromatischen Di-, Tri- und/oder Polycarbonsäuren mit Di-, Tri- und/oder Polyolen sowie die Polyesterole auf Lacton-Basis. Dazu zählen weiterhin Polyetherole, die z. B. durch Polymerisation von cyclischen Ethern oder durch Umsetzung von Alkylenoxiden mit einem Startermolekül erhältlich sind. Dazu zählen weiterhin auch übliche, dem Fachmann bekannte Polycarbonate mit endständigen Hydroxylgruppen, die durch Umsetzung der zuvor beschriebenen Diole oder auch von Bisphenolen, wie Bisphenol A, mit Phosgen oder Kohlensäurediestern erhältlich sind. Geeignet sind auch α,ω-Polyamidole, α,ω-Polymethyl(meth)acrylatdiole und/oder α,ω-Polybutyl(meth)acrylatdiole, wie z. B. MD-1000 und BD-1000 der Fa. Goldschmidt.

Die Herstellung von hyperverzweigten Polyesteramiden kann nach üblichen, dem Fachmann bekannten Verfahren erfolgen. In einer ersten Ausführungsform erfolgt die Herstellung von hyperverzeigten Polyesteramiden in einem einstufen Eintopfverfahren ausgehend von polyfunktionellen Aminoalkoholen und Dicarbonsäuren, wobei zumindest ein polyfunktioneller Aminoalkohol eingesetzt wird, der drei oder mehr als drei (z. B. 4, 5, 6 etc.) funktionelle Gruppen aufweist. Vorzugsweise liegt das molare Verhältnis von Dicarbonsäure zu Aminoalkohol in einem Bereich von 2:1 bis 1,1:1, besonders bevorzugt von 1,5:1 bis 1,2:1. Wenn in einer geeigneten Ausführungsform dieses einstufigen Verfahrens nur Dicarbonsäuren, d. h. Monomere vom Typ A₂ und trifunktionelle Aminoalkohole, d. h. Monomere vom Typ B₃, eingesetzt werden, so ist es zweckmäßig, die Reaktion zu unterbrechen, bevor der Gelpunkt erreicht ist. Zur Definition des Gelpunkts siehe Flory, Principles of Polymer Chemistry, Cornell University Press, 1953, S. 387 - 398. Der Gelpunkt kann sowohl nach der Theorie von Flory berechnet als auch durch Kontrolle der Viskosität des Reaktionsgemischs bestimmt werden. Es ist praktikabel, die Reaktion abzubrechen, sobald ein schneller Anstieg der Viskosität beobachtet wird.

In einer zweiten Ausführungsform erfolgt die Herstellung von hyperverzweigten Polyesteramiden ein einem zweistufigen Eintopfverfahren. Dabei wird in der ersten Stufe zunächst ein Präpolymer mit freien Carbonsäuregruppen hergestellt und dieses anschließend in einer zweiten Stufe mit polyfunktionellen Verbindungen umgesetzt, die zur Ester- bzw. Amidbildung befähigte funktionelle Gruppen aufweisen. In einer geeigneten Ausführung werden zur Herstellung der Präpolymere in der ersten Stufe die Carbonsäuren A₂ und Aminoalkohole B₃ eingesetzt. Das molare Verhältnis von Dicarbonsäure zu Aminoalkohol liegt vorzugsweise in einem Bereich von 2:1 bis 10:1, besonders bevorzugt von 2,5:1 bis 5:1 und insbesondere 2,7:1 bis 4:1. Bei dieser Vorgehensweise kann die Gelierung des Reaktionsgemischs im Allgemeinen auch bei höheren Umsatzraten leicht vermieden werden. Zur weiteren Umsetzung der Präpolymere in der zweiten Stufe können die zuvor genannten polyfunktionellen Amine, Aminoalkohole und Polyamine gegebenenfalls in Kombination mit weiteren Polycarbonsäuren eingesetzt werden. Auf geeignete und bevorzugte Ausführungsformen dieser Verbindungen wird auf das vorher Gesagte Bezug genommen. Vorzugsweise werden in der zweiten Reaktionsstufe überwiegend oder ausschließlich zweiwertige Verbindungen im Sinne einer Kettenverlängerung eingesetzt.

Vergleichbare Polymere wie sie nach dem zweistufigen Eintopfverfahren erhalten werden, können auch erhalten werden, wenn man die nach dem zuvor beschriebenen einstufigen Eintopfverfahren erhaltenen hyperverzweigten Polyesteramide einer anschließenden Modifizierung im Sinne einer polymeranalogen Umsetzung unterzieht, wobei für diese polymeranaloge Umsetzung dann die genannten polyfunktionellen Amine, Alkohole, Aminoalkohole und Carbonsäuren eingesetzt werden können. Möglich ist natürlich auch eine polymeranaloge Umsetzung sowohl der nach dem einstufigen als auch der nach dem zweistufigen Verfahren erhaltenen hyperverzweigten Polyesteramide mit monofunktionellen Verbindungen, z. B. Monoalkoholen, Monoaminen und Monocarbonsäuren, wie im Folgenden genauer beschrieben. Diese können zusätzliche funktionelle Gruppen zur weiteren Modifizierung der Polymereigenschaften aufweisen. Geeignete Abstopper sind beispielsweise Fettsäuren, Fettsäurederivate, wie Anhydride und Ester, Fettalkohole, Säuren und Säurederivate, die weitere funktionelle Gruppen aufweisen sowie Alkohole und Amine, die weitere funktionelle Gruppen aufweisen.

Die Veresterungs- und Amidierungsreaktion zur Herstellung von hyperverzweigten Polyesteramiden, wie auch die Amidierungsreaktion zur Herstellung von hyperverzweigten Polyamiden kann in Gegenwart wenigstens eines Katalysators erfolgen. Geeignete Katalysatoren sind beispielsweise saure Katalysatoren, organometallische Katalysatoren, Enzyme etc.

Geeignete saure Katalysatoren sind z. B. Schwefelsäure, Phosphorsäure, Phosphonsäure, hypophosphorige Säure, Aluminiumsulfathydrat, Alaun, saures Kieselgel und saures Aluminiumoxid. Geeignete Katalysatoren sind weiterhin aluminiumorganische Verbindungen der allgemeinen Formel Al(OR)₃ und titanorganische Verbindungen der allgemeinen Formel Ti(OR)₄, wobei die Reste R unabhängig voneinander für Alkyl, oder Cycloalkyl gemäß der eingangs gegebenen Definition stehen. Bevorzugte Reste R sind beispielsweise ausgewählt unter Isopropyl und 2-Ethylhexyl.

Bevorzugte saure metallorganische Katalysatoren sind beispielsweise ausgewählt unter Dialkylzinnoxiden der allgemeinen Formel R₂SnO, worin R unabhängig voneinander für Alkyl oder Cycloalkyl gemäß der eingangs gegebenen Definition steht. Dazu zählt vorzugsweise Di-n-butylzinnoxid, das als so genanntes Oxo-Zinn kommerziell erhältlich ist.

Geeignete saure organische Katalysatoren sind weiterhin saure organische Verbindungen, die wenigstens eine Säuregruppe, ausgewählt unter Phosphorsäuregruppen, Phosphonsäuregruppen, Sulfoxylgruppen, Sulfonsäuregruppen, etc. aufweisen. Bevorzugt ist beispielsweise p-Toluolsulfonsäure. Geeignete Katalysatoren sind weiterhin saurer Ionenaustauscher, beispielsweise mit Sulfonsäuregruppen modifizierte Polystyrolharze, die in üblicher Weise, z. B. mit Divinylbenzol, vernetzt sind.

### IV) Hyperverzweigte Polyesteramine

Im Rahmen der vorliegenden Erfindung bezeichnet der Ausdruck Polyesteramine ganz allgemein polymere Verbindungen, die in der Polymerkette Estergruppen und Aminogruppen aufweisen, wobei die Aminogruppen nicht Teil einer Amidgruppe sind. Zur Herstellung hyperverzweigter Polyesteramine können prinzipiell wenigstens zweiwertige Verbindungen eingesetzt werden, die eine vorzugsweise nicht mehr für eine Folgeumsetzung zur Verfügung stehende Aminogruppe sowie wenigstens zwei weitere zu einer Additions- oder Kondensationsreaktion befähigte funktionelle Gruppen aufweist. Dazu zählen beispielsweise N-(Alkyl)-N-(hydroxyalkyl)aminoalkyl-Carbonsäuren und -Carbonsäurederivate, N,N-Di(hydroxyalkyl)aminoalkyl-Carbonsäuren und -Carbonsäurederivate, N-(Alkyl)-N-(aminoalkyl)aminoalkyl-Carbonsäuren und -Carbonsäurederivate, N,N-Di(aminoalkyl)aminoalkyl-Carbonsäuren und -Carbonsäurederivate etc. Neben diesen Monomeren können die erfindungsgemäß eingesetzten hyperverzweigten Polyesteramine weitere polyfunktionelle Verbindungen eingebaut enthalten, die zwei oder mehr als zwei (z. B. 3, 4, 5, 6, etc.) funktionelle Gruppen aufweisen. Dazu zählen die zuvor beschriebenen Polycarbonsäuren, polyfunktionellen Amine, polyfunktionellen Alkohole, polyfunktionellen Aminoalkohole, worauf hier in vollem Umfang Bezug genommen wird.

Vorzugsweise erfolgt die Herstellung hyperverzweigter Polyesteramine unter Verwendung von AB₂- und/oder AB₃-Monomeren, die durch eine Umsetzung nach Art der Michael-Addition erhältlich sind.

In einer ersten Ausführung zur Herstellung eines AB₂-Monomers durch Michael-Addition wird ein Aminoalkohol, der eine sekundäre Aminogruppe und zwei Hydroxylgruppen aufweist, mit einer Verbindung mit aktivierter Doppelbindung, z. B. einer vinylogen Carbonylverbindung, umgesetzt.

Geeignete Amidoalkohole, die eine sekundäre Aminogruppe und zwei Hydroxylgruppen aufweisen, sind z. B. Diethanolamin, Dipropanolamin, Diisopropanolamin, 2-Amino-1,3-propandiol, 3-Amino-1,2-propandiol, Diisobutanolamin, Dicyclohexanolamin, etc.

Geeignete Verbindungen mit aktivierter Doppelbindung sind vorzugsweise ausgewählt unter Estern α,β-ethylenisch ungesättigter Mono- und Dicarbonsäuren mit einwertigen Alkoholen. Vorzugsweise sind die α,β-ethylenisch ungesättigten Mono- und Dicarbonsäuren ausgewählt unter Acrylsäure, Methacrylsäure, Fumarsäure, Maleinsäure, Itaconsäure, Crotonsäure, Maleinsäureanhydrid, Monobutylmaleat und Gemischen davon. Bevorzugt werden als Säurekomponente Acrylsäure, Methacrylsäure und deren Gemische eingesetzt. Bevorzugte vinyloge Verbindungen sind Methyl(meth)acrylat, Methylethacrylat, Ethyl(meth)acrylat, Ethylethacrylat, n-Butyl(meth)acrylat, tert.-Butyl(meth)acrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon. Besonders bevorzugt sind Methylacrylat und n-Butylacrylat.

In einer zweiten Ausführung zur Herstellung eines AB₂-Monomers durch Michael-Addition wird ein Aminoalkohol, der eine primäre Aminogruppe und eine Hydroxylgruppe aufweist, mit einer Verbindung mit einer Verbindung mit aktivierter Doppelbindung umgesetzt.

Geeignete Aminoalkohole, die eine primäre Aminogruppe und eine Hydroxylgruppe aufweisen, sind die zuvor zur Herstellung hyperverzweigter Polyesteramide genannten zweiwertigen Aminoalkohole, auf die hier in vollem Umfang Bezug genommen wird. Geeignete Verbindungen mit aktivierter Doppelbindung sind die zuvor bei der ersten Ausführungsform zur Herstellung eines AB₂-Monomers durch Michael-Addition genannten.

In einer dritten Ausführung zur Herstellung eines AB₃-Monomers durch Michael-Addition wird ein Aminoalkohol, der eine primäre Aminogruppe, eine sekundäre Aminogruppe und eine Hydroxylgruppe aufweist, mit drei aktivierten Doppelbindungen umgesetzt.

Ein geeigneter Aminoalkohol, der eine primäre Aminogruppe, eine sekundäre Aminogruppe und eine Hydroxylgruppe aufweist, ist Hydroxyethylethylendiamin. Geeignete Verbindungen mit aktivierter Doppelbindungen sind die zuvor bei der ersten Ausführung zur Herstellung eines AB₂-Monomers durch Michael-Addition genannten. Vorzugsweise erfolgt die Umsetzung nach Art der Michael-Addition in Substanz oder in einem unter den Reaktionsbedingungen inerten Lösungsmitteln. Geeignete Lösungsmittel sind z. B. höhersiedende Alkohole, wie Glycerin, aromatische Kohlenwasserstoffe, wie Benzol, Toluol, Xylol, etc. Die Umsetzung erfolgt vorzugsweise bei einer Temperatur im Bereich von 0 bis 100 °C, besonders bevorzugt 5 bis 80 °C und insbesondere 10 bis 70 °C. Vorzugsweise erfolgt die Reaktion in Gegenwart eines Inertgases, wie Stickstoff, Helium oder Argon und/oder in Gegenwart eines Radikalinhibitors. Allgemeine Arbeitsvorschriften zur Addition von Aminoalkoholen an aktivierte Doppelbindungen sind dem Fachmann bekannt. In einer bevorzugten Ausführungsform erfolgt die Herstellung der Monomere durch Michael-Addition und ihre anschließende Umsetzung in einer Polykondensation in Form einer Eintopfreaktion.

Die Herstellung der hyperverzweigten Polyesteramine aus den zuvor genannten oder aus anderen ABₓ-Monomeren erfolgt nach üblichen, dem Fachmann bekannten Verfahren. In einer geeigneten Vorgehensweise erfolgt die Herstellung von erfindungsgemäß geeigneten Polyesteraminen unter Verwendung der zuvor beschriebenen durch Michael-Addition erhältlichen AB₂-Monomere. Diese können zusätzlich in Gegenwart weiterer polyfunktioneller Monomere umgesetzt werden. Geeignete polyfunktionelle Monomere sind die zuvor bei der Herstellung hyperverzweigter Polyesteramide genannten polyfunktionellen Aminoalkohole, polyfunktionellen Amine, polyfunktionellen Alkohole und Polycarbonsäuren, worauf hier in vollem Umfang Bezug genommen wird. Gewünschtenfalls können zusätzlich Hydroxycarbonsäuren als Kettenverlängerer eingesetzt werden. Dazu zählen beispielsweise Milchsäure, Glykolsäure, etc.

In einer geeigneten Ausführung erfolgt die Herstellung der hyperverzweigten Polyesteramine in Gegenwart eines A₂B₂-Monomers. Dieses ist vorzugsweise ausgewählt unter 2-Amino-2-ethyl-1,3-propandiol, 2-Amino-2-methyl-1,3-propandiol, 1-Amino-2,3-propandiol, 2-Amino-1,3-propandiol, 2-Amino-1-phenyl-1,3-propandiol.

In einer weiteren geeigneten Ausführung erfolgt die Herstellung der hyperverzweigten Polyesteramine in Gegenwart eines so genannten Kernmoleküls. Geeignete Kernmoleküle sind beispielsweise Trimethylolpropan, Pentaerythrit, alkoxilierte Polyole, wie ethoxiliertes Trimethylolpropan, ethoxiliertes Glycerin, propoxiliertes Trimethylolpropan, propoxiliertes Glycerin, Polyamine, wie Tris(2-aminoethyl)amin, Ethylendiamin, Hexamethylendiamin, Diethanolamin, Diisopropanolamin, etc. Die Zugabe der kernbildenden Monomere kann zu Beginn oder im Verlauf der Reaktion erfolgen.

In einer weiteren geeigneten Ausführungsform erfolgt die Herstellung der hyperverzweigten Polyesteramine unter Verwendung eines aromatischen AB₂-Monomers. Geeignete aromatische AB₂-Monomere sind z. B. Amidol, Aminobenzylalkohol, 2-Amino-5-chlorbenzylalkohol, 2-Amino-9-fluorenol, etc.

Die Polykondensationsreaktion zur Herstellung von hyperverzweigten Polyesteraminen kann in Gegenwart eines Katalysators erfolgen. Geeignete Katalysatoren sind die zuvor zur Herstellung der hyperverzweigten Polyesteramiden beschriebenen Katalysatoren, worauf hier in vollem Umfang Bezug genommen wird. Geeignete Katalysatoren sind auch Enzyme, wie Lipasen oder Esterasen. Geeignete Lipasen oder Esterasen sind erhältlich aus Candida cylindracea, Candida lipolytica Candida rugosa, Candida antartica, Candidautilis, Chromobacterium viscosum, Geotrichum viscosum, Geotrichum candidum, Mucorjavanicus, Mucor mihei, Schweinepankreas, Pseudomonas spp., Pseudomonas fluorescens, Pseudomonas cepacia, Rhizopus arrhizus, Rhizopus delemar, Rhizopus niveus, Rhizopus oryzae, Aspergillus niger, Penicillium roquefortii, Penicillium camembertii, Esterasen aus Bacillus spp. und Bacillus thermoglucosidasius. Bevorzugte Enzyme sind Candida antartica Lipasen B und besonders bevorzugt immobilisierte Candida antartica Lipasen B, wie sie kommerziell von Novozymes Biotech Inc. unter der Bezeichnung Novozyme 435 erhältlich sind.

Vorteilhafterweise ist bei enzymatischer Katalyse die Umsetzung bei geringen Temperaturen in einem Bereich von etwa 40 bis 90 °C, bevorzugt 60 bis 70 °C, möglich. Vorzugsweise erfolgt die enzymatische Umsetzung in Gegenwart eines Inertgases, wie Kohlendioxid, Stickstoff, Argon, Helium.

Die zuvor beschriebenen hyperverzweigten Polymere können im Allgemeinen bereits als solche zur Herstellung wässriger Formulierungen von nicht oder nur gering löslichen Wirk- und Effektstoffen eingesetzt werden. In einer weiteren Ausführung werden die zuvor beschriebenen hyperverzweigten Polymere zusätzlich noch einer polymeranalogen Umsetzung unterzogen. So können die Polymereigenschaften in Abhängigkeit von Art und Menge der zur polymeranalogen Umsetzung eingesetzten Verbindungen gezielt für die jeweilige Anwendung angepasst werden. Geeignete modifizierte hyperverzweigte Polymere sind erhältlich ist durch polymeranaloge Umsetzung eines stickstoffhaltigen hyperverzweigten Polymers, das funktionelle Gruppen trägt, die zu einer Kondensations- oder Additionsreaktion befähigt sind, mit wenigstens einer Verbindung, die ausgewählt ist unter
a) Verbindungen, die wenigstens eine zu den zur Kondensations- oder Additionsreaktion befähigten Gruppen des hyperverzweigten Polymers komplementäre funktionelle Gruppe und zusätzlich wenigstens eine hydrophile Gruppe tragen,
b) Verbindungen, die wenigstens eine zu den zur Kondensations- oder Additionsreaktion befähigten Gruppen des hyperverzweigten Polymers komplementäre funktionelle Gruppe und zusätzlich wenigstens eine hydrophobe Gruppe tragen,
und Mischungen davon.

Als "komplementäre funktionelle Gruppen" wird im Rahmen der vorliegenden Erfindung ein Paar von funktionellen Gruppen verstanden, die in einer Reaktion, vorzugsweise einer Kondensations- oder Additionsreaktion, miteinander reagieren können. "Komplementäre Verbindungen" sind Paare von Verbindungen, die zueinander komplementäre funktionelle Gruppen aufweisen.

Bevorzugte komplementäre funktionelle Gruppen sind ausgewählt unter den komplementären funktionellen Gruppen a und b der nachfolgenden Übersicht, worin R und R' für organische Gruppen, wie Alkyl, bevorzugt C₁-C₂₀-Alkyl, wie Methyl, Ethyl, n-Propyl, Isopropyl, n-Butyl, tert.-Butyl, die isomeren Pentyle, Hexyle, Heptyle, Octyle etc.; Cycloalkyl, bevorzugt C₅-C₈-Cycloalkyl, insbesondere Cyclopentyl und Cyclohexyl; Aryl, bevorzugt Phenyl; Hetaryl etc. stehen und wobei R' auch Wasserstoff bedeuten kann.

### Übersicht: Beispiele komplementärer funktioneller Gruppen a) und b)

| Komponente A | Komponente B |
|---|---|
| Funktionelle Gruppe a) | Funktionelle Gruppe b) |
| -SH | -C(O)-OH, -NCO |
| -NH₂ | -C(O)-OR, NCO |
| -OH | -C(O)-O-C(O)-, -NCO, -COOH |
| -NHR | -NCO, -COOH |
| | -NH-C(O)-OR |
| | -NH-CH₂-OH |
| | -NH-CH₂-O-CH₃ |
| | -NH-C(O)-CH(-C(O)OR)(-C(O)-R) |
| | -NH-C(O)-CH(-C(O)OR)₂ |
| | -NH-C(O)-NR'₂ |
| | =Si(OR)₂ |
| | Epoxy |
| -C(O)-OH | Epoxy |
| -O-C(O)-CR'=CH₂ | -OH |
| -O-CR'=CH₂ | -NH₂ |
| | -C(O)-CH₂-C(O)-R |
| | -CH=CH₂ |

Zur Bildung von komplementären Paaren geeignete funktionelle Gruppen sind vorzugsweise ausgewählt unter Hydroxyl-, primären und sekundären Amino-, Thiol-, Carbonsäure-, Carbonsäureester-, Carbonsäureamid-, Carbonsäureanhydrid-, Sulfonsäure-, Sulfonsäureester-, Isocyanat-, verkappten Isocyanat-, Urethan-, Harnstoff-, Ether-und Expoxidgruppen.

Zur Reaktion geeignete Paare sind zum Beispiel einerseits Verbindungen mit aktiven Wasserstoffatomen, die z. B. ausgewählt sind unter Verbindungen mit Alkohol-, primären und sekundären Amin- und Thiolgruppen, und andererseits Verbindungen mit demgegenüber reaktiven Gruppen, die z. B. ausgewählt sind unter Carbonsäure-, Carbonsäureester-, Carbonsäureamid-, Carbonsäureanhydrid-, Isocyanat-, Urethan-, Harnstoff-, Alkohol-, Ether- und Epoxidgruppen. Ein weiteres geeignetes Paar umfasst z. B. Verbindungen mit Epoxidgruppen einerseits und Carbonsäuregruppen andererseits. Dabei ist es in der Regel unkritisch, welche Verbindung des Paares die Gruppe a) und welche die Gruppe b) trägt.

Bevorzugt werden zur polymeranalogen Umsetzung hydrophile Verbindungen eingesetzt. Geeignete hydrophile Gruppen sind ausgewählt unter ionogenen, ionischen und nichtionischen hydrophilen Gruppen. Bei den ionogenen bzw. ionischen Gruppen handelt es sich vorzugsweise um Carbonsäuregruppen und/oder Sulfonsäuregruppen und/oder stickstoffhaltige Gruppen (Amine) bzw. Carboxylatgruppen und/oder Sulfonatgruppen und/oder quaternisierte oder protonierte Gruppen. Verbindungen, die Säuregruppen enthalten, können durch teilweise oder vollständige Neutralisation in die entsprechenden Salze überführt werden. Geeignete Basen für die Neutralisation sind beispielsweise Alkalimetallbasen, wie Natronlauge, Kalilauge, Natriumcarbonat, Natriumhydrogencarbonat, Kaliumcarbonat oder Kaliumhydrogencarbonat und Erdalkalimetallbasen, wie Calciumhydroxid, Calciumoxid, Magnesiumhydroxid oder Magnesiumcarbonat sowie Ammoniak und Amine, wie Trimethylamin, Triethylamin etc. Aus Verbindungen mit Aminstickstoffatomen lassen sich geladene kationische Gruppen entweder durch Protonierung, z. B. mit Carbonsäuren, wie Essigsäure, oder durch Quaternisierung, z. B. mit Alkylierungsmitteln, wie C₁-C₄-Alkylhalogeniden oder -sulfaten, erzeugen. Beispiele solcher Alkylierungsmittel sind Ethylchlorid, Ethylbromid, Dimethylsulfat und Diethylsulfat.

Durch polymeranaloge Umsetzung erhältliche hyperverzweigte Polymere mit ionischen hydrophilen Gruppen sind in der Regel wasserlöslich.

Vorzugsweise werden zur polymeranalogen Umsetzung Hydroxycarbonsäuren, wie Hydroxyessigsäure (Glykolsäure), Hydroxypropionsäure (Milchsäure), Hydroxybernsteinsäure (Äpfelsäure), Hydroxypivalinsäure, 4-Hydroxybenzoesäure, 12-Hydroxydodecansäure, Dimethylolpropionsäure etc. eingesetzt.

Des Weiteren bevorzugt werden zur polymeranalogen Umsetzung Hydroxysulfonsäuren, wie Hydroxymethansulfonsäure oder 2-Hydroxyethansulfonsäure, eingesetzt.

Des Weiteren bevorzugt werden zur polymeranalogen Umsetzung Mercaptocarbonsäuren, wie Mercaptoessigsäure, eingesetzt.

Des Weiteren bevorzugt werden zur polymeranalogen Umsetzung Aminosulfonsäuren der Formel:

R¹HN-Y-SO₃H

eingesetzt, worin
- Y: für o-, m- oder p-Phenylen oder geradkettiges oder verzweigtes C₂-C₆-Alkylen steht, das gegebenenfalls durch 1, 2 oder 3 Hydroxygruppen substituiert ist, und
- R¹: für ein Wasserstoffatom, eine C₁-C₁₂-Alkylgruppe (vorzugsweise C₁-C₁₀- und ins- besondere C₁-C₆-Alkylgruppe) oder eine C₅-C₆-Cycloalkylgruppe steht, wobei die Alkylgruppe oder die Cycloalkylgruppe gegebenenfalls durch 1, 2 oder 3 Hydro- xygruppen, Carboxylgruppen oder Sulfonsäuregruppen substituiert sein kann.

Bevorzugt handelt es sich bei den Aminosulfonsäuren der obigen Formel um Taurin, N-(1,1-Dimethyl-2-hydroxyethyl)-3-amino-2-hydroxypropansulfonsäure oder 2-Aminoethylaminoethansulfonsäure.

Des Weiteren bevorzugt werden zur polymeranalogen Umsetzung α-, β- oder γ-Aminosäuren, beispielsweise Glycin, Alanin, Valin, Leucin, Isoleucin, Phenylalanin, Thyrosin, Prolin, Hydroxyprolin, Serin, Threonin, Methionin, Cystein, Tryptophan, β-Alanin, Asparaginsäure oder Glutaminsäure, eingesetzt.

Des Weiteren bevorzugt werden zur polymeranalogen Umsetzung Polyetherole eingesetzt. Geeignete Polyetherole sind lineare oder verzweigte endständige Hydroxylgruppen aufweisende Substanzen, die Etherbindungen enthalten und ein Molekulargewicht im Bereich von z. B. etwa 300 bis 10000 aufweisen. Dazu zählen beispielsweise Polyalkylenglykole, z. B. Polyethylenglykole, Polypropylenglykole, Polytetrahydrofurane, Copolymerisate aus Ethylenoxid, Propylenoxid und/oder Butylenoxid, die die Alkylenoxideinheiten statistisch verteilt oder in Form von Blöcken einpolymerisiert enthalten. Geeignet sind auch α,ω-Diaminopolyether, die durch Aminierung von Polyetherolen mit Ammoniak herstellbar sind. Derartige Verbindungen sind kommerziell unter der Bezeichnung Jeffamine® erhältlich.

Des Weiteren werden zur polymeranalogen Umsetzung Diamine, Polyamine und Mischungen davon eingesetzt.

Geeignete polyfunktionelle Amine, Alkohole und Aminoalkohole sind die zuvor genannte.

Zur polymeranalogen Umsetzung geeignete hydrophobe Gruppen sind vorzugsweise ausgewählt unter gesättigten oder ungesättigten Kohlenwasserstoffresten mit 8 bis 40, bevorzugt 9 bis 35, insbesondere 10 bis 30 Kohlenstoffatomen. Bevorzugt handelt es sich um Alkyl-, Alkenyl-, Cycloalkyl- oder Arylreste. Die Cycloalkyl- oder Arylreste können 1, 2 oder 3 Substituenten, vorzugsweise Alkyl- oder Alkenylsubstituenten, aufweisen. Im Rahmen der vorliegenden Erfindung werden mit "Alkenylresten" Reste bezeichnet, die eine, zwei oder mehrere Kohlenstoff-Kohlenstoff-Doppelbindungen aufweisen.

Im Rahmen der vorliegenden Erfindung umfasst der Ausdruck C₈-C₄₀-Alkyl geradkettige und verzweigte Alkylgruppen. Vorzugsweise handelt es sich dabei um geradkettige und verzweigte C₉-C₃₅-Alkyl-, besonders bevorzugt um C₁₀-C₃₀- und speziell C₁₂-C₂₆-Alkylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen. Dazu zählen insbesondere n-Octyl, Ethylhexyl, 1,1,3,3-Tetramethylbutyl, n-Nonyl, n-Decyl, n-Undecyl, n-Dodecyl, n-Tridecyl, Myristyl, Pentadecyl, Palmityl (=Cetyl), Heptadecyl, Octadecyl, Nonadecyl, Arrachinyl, Behenyl, Lignocerenyl, Cerotinyl, Melissinyl etc.

C₈-C₄₀-Alkenyl steht vorzugsweise für geradkettige und verzweigte Alkenylgruppen, die einfach, zweifach oder mehrfach ungesättigt sein können. Bevorzugt handelt es sich um C₉-C₃₅-, insbesondere um C₁₀-C₃₀- und speziell um C₁₂-C₂₆-Alkenylgruppen. Dazu zählen insbesondere Octenyl, Nonenyl, Decenyl, Undecenyl, Dodecenyl, Tridecenyl, Tetradecenyl, Pentadecenyl, Hexadecenyl, Heptadecenyl, Octadecenyl, Nonadecenyl, Linolylyl, Linolenylyl, Eleostearyl etc. und insbesondere Oleyl (9-Octadecenyl).

Bevorzugte Verbindungen zur hydrophoben polymeranalogen Umsetzung sind 1-Nonylamin, 1-Decylamin, 1-Undecylamin, 1-Undec-10-enylamin, 1-Tridecylamin, 1-Tetradecylamin, 1-Pentadecylamin, 1-Hexadecylamin, 1-Heptadecylamin, 1-Octadecylamin, 1-Octadeca-9,12-dienylamin, 1-Nonadecylamin, 1-Eicosylamin, 1-Eicos-9-enylamin, 1-Heneicosylamin, 1-Docosylamin und insbesondere Oleylamin und 1-Hexadecylamin (Cetylamin) oder aus natürlich vorkommenden Fettsäuren hergestellte Amingemische, wie z. B. Talgfettamine, die überwiegend gesättigte und ungesättigte C₁₄-, C₁₆-C₁₈-Alkylamine enthalten oder Kokosamine, die gesättigte, einfach und zweifach ungesättigte C₈-C₂₂-, vorzugsweise C₁₂-C₁₄-Alkylamine enthalten.

Des Weiteren bevorzugt ist die Verbindung zur polymeranalogen Umsetzung ausgewählt unter einwertigen Alkoholen, die einen der zuvor genannten hydrophoben Reste aufweisen. Solche Alkohole und Alkoholgemische sind z. B. erhältlich durch Hydrierung von Fettsäuren aus natürlichen Fetten und Ölen oder von synthetischen Fettsäuren, z. B. aus der katalytischen Oxidation von Paraffinen. Geeignete Alkohole und Alkoholgemische sind weiterhin erhältlich durch Hydroformylierung von Olefinen mit gleichzeitiger Hydrierung der Aldehyde, wobei im Allgemeinen Gemische aus geradkettigen und verzweigten primären Alkoholen (Oxo-Alkohole) resultieren. Geeignete Alkohole und Alkoholgemische b) sind weiterhin erhältlich durch partielle Oxidation von n-Paraffinen nach bekannten Verfahren, wobei überwiegend lineare sekundäre Alkohole erhalten werden. Geeignet sind weiterhin die durch aluminiumorganische Synthese erhältlichen, im Wesentlichen primären, geradkettigen und geradzahligen Ziegler-Alkohole.

Geeignete sind auch Amine mit einer primären oder sekundären Aminogruppe, wie z. B. Methylamin, Ethylamin, n-Propylamin, Isopropylamin, Dimethylamin, Diethylamin, Di-n-propylamin, Diisopropylamin etc.

Zur polymeranalogen Umsetzung geeignete einwertige Alkohole sind z. B. monofunktionelle Alkohole, wie z. B. Methanol, Ethanol, n-Propanol, Isopropanol, Octanol, Nonanol, Decanol, Undecanol, Dodecanol, Tridecanol, Tetradecanol, Pentadecanol, Hexadecanol, Heptadecanol, Octadecanol, etc. und Mischungen davon. Des Weiteren kann es sich um einwertige Polyetheralkohole mit einem zahlenmittleren Molgewicht im Bereich von etwa 500 bis 10000 g/mol, vorzugsweise von 1000 bis 5000 g/mol, handeln. Einwertige Polyetheralkohole sind durch Alkoxylierung von einwertigen Startermolekülen, wie beispielsweise Methanol, Ethanol oder n-Butanol, erhältlich, wobei als Alkoxylierungsmittel Ethylenoxid oder Gemische von Ethylenoxid mit anderen Alkylenoxiden, besonders Propylenoxid, eingesetzt werden.

Zur polymeranalogen Umsetzung geeignete Monoisocyanate sind z. B. C₈-C₄₀-Alkylisocyanate, die aus den zuvor genannten Aminen und Amingemischen durch Phosgenierung oder aus natürlichen oder synthetischen Fettsäuren und Fettsäuregemischen durch Hofmann-, Curtius- oder Lossen-Abbau erhältlich sind.

Die zuvor genannten Verbindungen zur polymeranalogen Umsetzung können jeweils einzeln, als Mischungen aus ausschließlich hydrophilen Verbindungen oder aus ausschließlich hydrophoben Verbindungen sowie als Mischungen von hydrophilen Verbindungen mit hydrophoben Verbindungen eingesetzt werden. Durch polymeranaloge Umsetzung von Urethan- und/oder Harnstoffgruppen tragenden hyperverzweigten Polymeren mit einzelnen Verbindungen oder mit Gemischen davon lassen sich die Eigenschaften der hyperverzweigten Polymere in einem weiten Bereich variieren.

Im Folgenden werden einige weitere Ausführungsformen für polymeranaloge Umsetzungen aufgezeigt:
Durch Umsetzung mit Acrylatgruppen enthaltenden Verbindungen, wie beispielsweise Acrylatgruppen enthaltenden Alkoholen, wie 2-Hydroxyethylacrylat oder 2-Hydroxyethylmethacrylat, lassen sich hyperverzweigte Polymere erhalten, die polymerisierbare olefinische Gruppen aufweisen und die zur Herstellung von strahlungsvernetzenden, insbesondere von UV-vernetzenden Polymeren, eingesetzt werden können. Durch Umsetzung mit entsprechend substituierten Alkoholen lassen sich auch Epoxid- oder Vinylethergruppen einbringen, die für kationisch vernetzende Polymere genutzt werden können.

Oxidativ trocknende hyperverzweigte Polymere können erhalten werden, indem man NCO- oder Urethangruppen enthaltende Polymere mit einfach oder mehrfach ungesättigten Fettsäureestern, die mindestens eine OH-Gruppe aufweisen, oder mit einfach oder mehrfach ungesättigten Fettalkoholen oder Fettaminen, insbesondere mit 3 bis 40 Kohlenstoffatomen, umsetzt. Beispielsweise können OH-Gruppen enthaltende Ester der Linolsäure, Linolensäure oder Eleostearinsäure mit NCO-Gruppen umgesetzt werden. Weiterhin können NCO- oder Urethangruppen aber auch direkt mit Vinyl- oder Allyl-Gruppen enthaltenden Alkoholen oder Aminen umgesetzt werden.

Zur Herstellung von hyperverzweigten Polymere, die verschiedenartige Funktionalitäten aufweisen, kann man beispielsweise 2 Mol 2,4-TDI mit einer Mischung aus 1 Mol Trimethylolpropan und 1 Mol Dimethylolpropionsäure reagieren lassen. Hierbei wird ein Produkt erhalten, das sowohl über Carbonsäuregruppen als auch über OH-Gruppen verfügt.

Weiterhin können solche Produkte auch dadurch erhalten werden, dass man mit einem ABₓ-Molekül polymerisiert, die Polymerisation bei dem gewünschten Umsetzungsgrad abbricht und anschließend nur einen Teil der ursprünglich vorhandenen funktionellen Gruppen, beispielsweise nur einen Teil der OH- oder der NCO-Gruppen, umsetzt. Beispielsweise kann man so bei einem NCO-terminierten Polymer aus 2,4-TDI und Glycerin einen Teil der NCO-Gruppen mit Ethanolamin und die übrigen NCO-Gruppen mit Mercaptoessigsäure umsetzen.

Weiterhin kann man ein OH-terminiertes Polymerisat aus Isophorondiisocyanat und Diethanolamin nachträglich hydrophobieren, indem man zum Beispiel einen Teil der OH-Gruppen mit Dodecylisocyanat oder mit Dodecansäure umsetzt. Die Umfunktionalisierung eines hyperverzweigten Polyurethans oder die Anpassung der Polymereigenschaften an das Anwendungsproblem kann vorteilhaft unmittelbar im Anschluss an die Polymerisationsreaktion erfolgen, ohne dass das NCO-terminierte Polyurethan vorher isoliert wird. Die Funktionalisierung kann aber auch in einer separaten Reaktion erfolgen.

Die erfindungsgemäß eingesetzten hyperverzweigten Polymere weisen im Regelfall eine mittlere Anzahl an funktionellen Gruppen von mindestens 4 auf. Die Zahl der funktionellen Gruppen ist prinzipiell nicht nach oben beschränkt. Im Allgemeinen weisen die erfindungsgemäß verwendeten hyperverzweigten Polymere jedoch nicht mehr als 100 funktionelle Gruppen auf. Bevorzugt weisen die hyperverzweigten Polymere 4 bis 30, besonders bevorzugt 5 bis 20 funktionelle Gruppen auf. Vorzugsweise liegt das zahlenmittlere Molekulargewicht Mₙ in einem Bereich von 400 bis 100000 g/mol, besonders bevorzugt von 500 bis 80000 g/mol.

Das gewichtsmittlere Molekulargewicht M_{w} liegt vorzugsweise in einem Bereich von 500 bis 500000 g/mol, besonders bevorzugt 1000 bis 100000 g/mol.

Die Polydispersität (M_{w}/Mₙ) liegt vorzugsweise in einem Bereich von 1,1 bis 50, besonders bevorzugt von 1,3 bis 45.

Die hyperverzweigten Polymere können in Mischung oder in Kombination mit oberflächenaktiven Substanzen, wie z. B. anionischen, kationischen, zwitterionischen oder nicht ionischen Tensiden bzw. Netzmitteln eingesetzt werden. Sie können weiterhin in Kombination mit weiteren Polymeren eingesetzt werden, wobei u. U. eine Verstärkung der solubilisierenden Wirkung erzielt werden kann.

Die Herstellung der erfindungsgemäßen Wirkstoffzusammensetzung kann in unterschiedlicher Weise erfolgen:

In einer ersten Ausführungsform der vorliegenden Erfindung stellt man die wässrige Wirkstoffzusammensetzung her, indem man zunächst eine homogene, nicht wässrige Mischung, bestehend aus hyperverzweigtem Polymer und Wirkstoff und/oder Effektstoff herstellt und die so erhaltene Mischung anschließend in Wasser oder einem wässrigen Medium dispergiert. Zum Herstellen der homogenen, nicht wässrigen Mischung wird man in der Regel den Wirkstoff in eine flüssige Form der hyperverzweigten Polymerzusammensetzung, beispielsweise eine Schmelze oder vorzugsweise eine Lösung in einem organischen Lösungsmittel einarbeiten. Sofern man ein Lösungsmittel verwendet, wird man anschließend das Lösungsmittel möglichst weitgehend und vorzugsweise vollständig entfernen, wobei man eine feste Lösung des Wirkstoffs in der hyperverzweigten Polymerzusammensetzung erhält. Geeignete Lösungsmittel hierfür sind grundsätzlich solche, die sowohl den Wirkstoff als auch das Polymer zu lösen vermögen, beispielsweise aliphatische Nitrile wie Acetonitril und Propionitril, N,N-Dialkylamide aliphatischer Carbonsäuren wie Dimethylformamid und Dimethylacetamid, N-Alkyllactame wie N-Methylpyrrolidon, die vorgenannten aliphatischen und alicyclischen Ether, beispielsweise Tetrahydrofuran, halogenierte Kohlenwasserstoffe wie Dichlormethan, Dichlorethan und Mischungen der vorgenannten Lösungsmittel. Zur Herstellung der erfindungsgemäßen wässrigen Zusammensetzung wird man anschließend die so erhaltene feste Lösung des Wirkstoffs in der hyperverzweigten Polymerzusammensetzung in einem wässrigen Medium durch Rühren dispergieren. Das Rühren kann bei Temperaturen im Bereich der Umgebungstemperatur als auch bei erhöhter Temperatur erfolgen, beispielsweise bei einer Temperatur im Bereich von 10 bis 80 °C und insbesondere im Bereich von 20 bis 50 °C.

In einer zweiten Ausführungsform der vorliegenden Erfindung erfolgt die Herstellung der wässrigen Wirkstoffzusammensetzung durch Einarbeiten des Wirkstoffs und/oder Effektstoffs in eine wässrige Lösung/Dispersion der hyperverzweigten Polymerzusammensetzung. Hierzu geht man in der Regel so vor, das man das Einarbeiten bei einer Temperatur durchführt, die oberhalb der Schmelztemperatur des Wirk- bzw. Effektstoffs liegt und vorzugsweise bei einer Temperatur, bei der die Wirk- bzw. Effektstoffschmelze niedrigviskos ist, d. h. eine Viskosität im Bereich von 1 bis 1000 mPa.s (nach DIN 53019-2 bei 25 °C) aufweist. Vorzugsweise erfolgt das Einarbeiten unter Anwendung von starken Scherkräften, beispielsweise in einem Ultraturrax.

In einer dritten Ausführungsform der Erfindung erfolgt die Herstellung der wässrigen Wirkstoffzusammensetzung durch ein Verfahren umfassend die folgenden Schritte a) bis c):
a) Herstellung einer Lösung von Wirkstoff und/oder Effektstoff und gegebenenfalls hyperverzweigten Polymerzusammensetzung in einem organischen Lösungsmittel, das einen Siedepunkt unter dem von Wasser aufweist und
b) Vermischen der Lösung des Wirkstoffs und/oder Effektstoffs mit Wasser oder mit einer wässrigen Lösung des hyperverzweigten Polymers und
c) Entfernen des Lösungsmittels.

Hierbei kann man alternativ so vorgehen, dass die Lösung des Wirkstoffs die hyperverzweigte Polymerzusammensetzung enthält und man diese Lösung mit Wasser vermischt oder dass die Lösung des Wirkstoffs nur einen Teil der hyperverzweigten Polymerzusammensetzung oder keine hyperverzweigte Polymerzusammensetzung enthält und man diese Lösung mit einer wässrigen Lösung oder Dispersion der hyperverzweigten Polymerzusammensetzung vermischt. Das Vermischen kann in geeigneten Rührgefäßen erfolgen, wobei manentweder Wasser oder die wässrige Lösung der hyperverzweigten Polymerzusammensetzung vorlegen kann und hierzu die Lösung des Wirk- oder Effektstoffs gibt, oder alternativ die Lösung des Wirk- oder Effektstoffs vorlegt und hierzu das Wasser bzw. die wässrige Lösung der hyperverzweigten Polymerzusammensetzung gibt. Anschließend entfernt man das organische Lösungsmittel, z. B. durch Destillation, wobei man gegebenenfalls Wasser zusetzt.

In einer bevorzugten Variante dieser Ausführungsform gibt man die Wirkstofflösung und das Wasser bzw. die wässrige Lösung der hyperverzweigten Polymerzusammensetzung kontinuierlich in eine Mischzone und entnimmt dieser kontinuierlich die Mischung, aus der man anschließend das Lösungsmittel entfernt. Die Mischzone kann beliebig ausgestaltet werden. Grundsätzlich sind hierfür alle Apparaturen geeignet, die ein kontinuierliches Mischen von Flüssigkeitsströmen ermöglichen. Derartige Apparaturen sind bekannt, z. B. aus Continuous Mixing of Fluids (J.-H. Henzler) in Ullmann's Encyclopedia, 5th ed. on CD-Rom, Wiley-VCH. Die Mischzone können als statische oder dynamische Mischer oder Mischformen davon ausgestaltet sein. Als Mischzonen kommen insbesondere auch Jet-Mischer oder vergleichbare Mischer mit Düsen in Betracht. In einer bevorzugten Ausgestaltung handelt es sich bei der Mischzone um die im "Handbook of Industrial Crystallization" (A. S. Myerson, 1993 Butterworth-Heinemann, Seite 139, ISBN 0-7506-9155-7) beschriebene Apparatur oder eine vergleichbare Apparatur.

Das Volumenverhältnis von Wirkstofflösung zu Wasser bzw. wässriger Lösung der hyperverzweigten Polymerzusammensetzung kann über weite Bereich variiert werden und liegt vorzugsweise im Bereich von 10:1 bis 1:20 und insbesondere im Bereich von 5:1 bis 1:10.

Naturgemäß sollte das Lösungsmittel geeignet sein, die hyperverzweigte Polymerzusammensetzung und den Wirkstoff in den gewünschten Mengenverhältnissen zu lösen. Geeignete Lösungsmittel kann der Fachmann durch Routineexperimente ermitteln. Beispiele für geeignete Lösungsmittel sind C₂-C₄-Alkanole wie Ethanol, n-Propanol, n-Butanol, Isobutanol, die vorgenannten aliphatischen und alicyclischen Ether wie Diethylether, Diisopropylether, Methyl-tert.-butylether, Dioxan, Tetrahydrofuran, Ketone wie Aceton, Methylethylketon.

In den erfindungsgemäßen wässrigen Wirkstoffzusammensetzungen hat es sich als vorteilhaft erwiesen, wenn das Gewichtsverhältnis von Wirkstoff und/oder Effektstoff zu hyperverzweigtem Polymer im Bereich von 1:10 bis 3:1 und insbesondere im Bereich von 1:5 bis 2:1 liegt.

Der Gehalt an Wirk- und/oder Effektstoff kann über weite Bereiche variiert werden. Insbesondere ermöglichen die erfindungsgemäß eingesetzten hyperverzweigten Polymere das Herstellen von so genannten Wirkstoffkonzentraten, welche den Wirkstoff in einer Menge von wenigstens 5 Gew.-%, bezogen auf das Gesamtgewicht der Zusammensetzung, enthalten.

Vorteilhafterweise können die erfindungsgemäßen wässrigen Wirkstoffzusammensetzungen lösungsmittelfrei oder lösungsmittelarm formuliert werden, d. h. der Anteil an flüchtigen Bestandteilen in der wässrigen Wirkstoffzusammensetzung beträgt häufig nicht mehr als 10 Gew.-%, insbesondere nicht mehr als 5 Gew.-% und speziell nicht mehr als 1 Gew.-%, bezogen auf das Gesamtgewicht der Zusammensetzung. Flüchtige Bestandteile sind dabei solche, die bei Normaldruck einen Siedepunkt unterhalb 200 °C aufweisen.

Gegenstand der Erfindung sind auch die durch Trocknen der wässrigen Wirkstoffzusammensetzungen erhältlichen Feststoffe, z. B. Pulver. Die Herstellung kann nach üblichen, dem Fachmann bekannten Trocknungsverfahren, z. B. durch Sprühtrocknen, Walzentrocknen oder Gefriertrocknen erfolgen.

In den erfindungsgemäßen wässrigen Zusammensetzungen können eine Vielzahl unterschiedlicher Wirk- und Effektstoffe formuliert werden. Eine besondere Ausführungsform der Erfindung betrifft die Formulierung von Wirkstoffen für den Pflanzenschutz, d. h. von Herbiziden, Fungiziden, Nematiziden, Akariziden, Insektiziden sowie Wirkstoffen, die das Pflanzenwachstum regulieren. Gegenstand der Erfindung ist daher auch ein Pflanzenschutzmittel, enthaltend
A) wenigstens ein stickstoffatomhaltiges hyperverzweigtes Polymer, wie zuvor definiert,
B) wenigstens einen Wirkstoff für den Pflanzenschutz, der in Wasser bei 25 °C und 1013 mbar eine Löslichkeit unterhalb 10 g/l aufweist, und
C) gegebenenfalls wenigstens einen weiteren, von B) verschiedenen Wirkstoff für den Pflanzenschutz und/oder wenigstens einen Hilfsstoff.

Beispiele für fungizide Wirkstoffe, die als erfindungsgemäße wässrige Wirkstoffzusammensetzung formuliert werden können, umfassen:
- Acylalanine wie Benalaxyl, Metalaxyl, Ofurace, Oxadixyl;
- Aminderivate wie Aldimorph, Dodine, Dodemorph, Fenpropimorph, Fenpropidin, Guazatine, Iminoctadine, Spiroxamin, Tridemorph;
- Anilinopyrimidine wie Pyrimethanil, Mepanipyrim oder Cyrodinyl;
- Antibiotika wie Cycloheximid, Griseofulvin, Kasugamycin, Natamycin, Polyoxin und Streptomycin;
- Azole wie Bitertanol, Bromoconazol, Cyproconazole, Difenoconazol, Dinitroconazol, Epoxiconazol, Fenbuconazol, Fluquiconazol, Flusilazol, Flutriafol, Hexaconazol, Imazalil, Ipconazol, Metconazol, Myclobutanil, Penconazol, Propiconazol, Prochloraz, Prothioconazol, Tebuconazol, Tetraconazol, Triadimefon, Triadimenol, Triflumizol, Triticonazol;
- 2-Methoxybenzophenone, wie sie in EP-A 897904 durch die allgemeine Formel I beschrieben werden, z. B. Metrafenon;
- Dicarboximide wie Iprodion, Myclozolin, Procymidon, Vinclozolin;
- Dithiocarbamate wie Ferbam, Nabam, Maneb, Mancozeb, Metam, Metiram, Propineb, Polycarbamat, Thiram, Ziram, Zineb;
- Heterocyclische Verbindungen wie Anilazine, Benomyl, Boscalid, Carbendazim, Carboxin, Oxycarboxin, Cyazofamid, Dazomet, Dithianon, Famoxadon, Fenamidon, Fenarimol, Fuberidazole, Flutolanil, Furametpyr, Isoprothiolane, Mepronil, Nuarimol, Picobezamid, Probenazole, Proquinazid, Pyrifenox, Pyroquilon, Quinoxyfen, Silthiofam; Thiabendazole, Thifluzamid, Thiophanatmethyl, Tiadinil, Tricyclazole, Triforine;
- Nitrophenylderivative wie Binapacryl, Dinocap, Dinobuton, Nitrophthalisopropyl;
- Phenylpyrrole wie Fenpiclonil sowie Fludioxonil;
- nicht klassifizierte Fungizide wie Acibenzolar-S-methyl, Benthiavalicarb, Carpropamid, Chlorothalonil, Cyflufenamid, Cymoxanil, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminum, Iprovalicarb, Hexachlorobenzol, Metrafenon, Pencycuron, Propamocarb, Phthalide, Toloclofos-Methyl, Quintozene, Zoxamid;
- Strobilurine wie sie in WO 03/075663 durch die allgemeine Formel I beschrieben werden, beispielsweise Azoxystrobin, Dimoxystrobin, Fluoxastrobin, Kresoxim-methyl, Metominostrobin, Orysastrobin, Picoxystrobin, Pyraclostrobin sowie Trifloxystrobin;
- Sulfensäure-Derivative wie Captafol, Captan, Dichlofluanid, Folpet, Tolylfluanid;
- Zimtsäureamide und Analoga wie Dimethomorph, Flumetover, Flumorp;
- 6-Aryl-[1,2,4]triazolo[1,5-a]pyrimidine wie sie z. B. in WO 98/46608, WO 99,41255 oder WO 03/004465 jeweils durch die allgemeine Formel I beschrieben werden;
- Amidfungizide wie Cyclofenamid sowie (Z)-N-[α-(Cyclopropylmethoxyimino)-2,3-difluoro-6-(difluoromethoxy)benzyl]-2-phenylacetamid.

Beispiele für Herbizide, die als erfindungsgemäße wässrige Wirkstoffzusammensetzung formuliert werden können, umfassen:
- 1,3,4-Thiadiazole wie Buthidazole und Cyprazole;
- Amide wie Allidochlor, Benzoylpropethyl, Bromobutide, Chlorthiamid, Dimepiperate, Dimethenamid, Diphenamid, Etobenzanid, Flampropmethyl, Fosamin, Isoxaben, Metazachlor, Monalide, Naptalame, Pronamid, Propanil;
- Aminophosphorsäuren wie Bilanafos, Buminafos, Glufosinateammonium, Glyphosate, Sulfosate;
- Aminotriazole wie Amitrol, Anilide wie Anilofos, Mefenacet;
- Aryloxyalkansäure wie 2,4-D, 2,4-DB, Clomeprop, Dichlorprop, Dichlorprop-P, Dichlorprop-P, Fenoprop, Fluroxypyr, MCPA, MCPB, Mecoprop, Mecoprop-P, Napropamide, Napropanilide, Triclopyr;
- Benzoesäuren wie Chloramben, Dicamba;
- Benzothiadiazinone wie Bentazon;
- Bleacher wie Clomazone, Diflufenican, Fluorochloridone, Flupoxam, Fluridone, Pyrazolate, Sulcotrione;
- Carbamate wie Carbetamid, Chlorbufam, Chlorpropham, Desmedipham, Phenmedipham, Vernolate;
- Chinolinsäuren wie Quinclorac, Quinmerac;
- Dichlorpropionsäuren wie Dalapon;
- Dihydrobenzofurane wie Ethofumesate;
- Dihydrofuran-3-on wie Flurtamone;
- Dinitroaniline wie Benefin, Butralin, Dinitramin, Ethalfluralin, Fluchloralin, Isopropalin, Nitralin, Oryzalin, Pendimethalin, Prodiamine, Profluralin, Trifluralin, Dinitrophenole wie Bromofenoxim, Dinoseb, Dinosebacetat, Dinoterb, DNOC, Minoterbacetat;
- Diphenylether wie Acifluorfensodium, Aclonifen, Bifenox, Chlornitrofen, Difenoxuron, Ethoxyfen, Fluorodifen, Fluoroglycofenethyl, Fomesafen, Furyloxyfen, Lactofen, Nitrofen, Nitrofluorfen, Oxyfluorfen;
- Dipyridyle wie Cyperquat, Difenzoquatmethylsulfat, Diquat, Paraquatdichlorid;
- Imidazole wie Isocarbamid;
- Imidazolinone wie Imazamethapyr, Imazapyr, Imazaquin, Imazethabenzmethyl, Imazethapyr, Imazapic, Imazamox;
- Oxadiazole wie Methazole, Oxadiargyl, Oxadiazon;
- Oxirane wie Tridiphane;
- Phenole wie Bromoxynil, loxynil;
- Phenoxyphenoxypropionsäureester wie Clodinafop, Cyhalofopbutyl, Diclofopmethyl, Fenoxapropethyl, Fenoxaprop-p-ethyl, Fenthiapropethyl, Fluazifopbutyl, Fluazifop-p-butyl, Haloxyfopethoxyethyl, Haloxyfopmethyl, Haloxyfop-p-methyl,

Isoxapyrifop, Propaquizafop, Quizalofopethyl, Quizalofop-p-ethyl, Quizalofoptefuryl;
- Phenylessigsäuren wie Chlorfenac;
- Phenylpropionsäuren wie Chlorophenpropmethyl;
- ppi-Wirkstoffe (ppi = preplant incorporated) wie Benzofenap, Flumicloracpentyl, Flumioxazin, Flumipropyn, Flupropacil, Pyrazoxyfen, Sulfentrazone, Thidiazimin;
- Pyrazole wie Nipyraclofen;
- Pyridazine wie Chloridazon, Maleic hydrazide, Norflurazon, Pyridate;
- Pyridincarbonsäuren wie Clopyralid, Dithiopyr, Picloram, Thiazopyr;
- Pyrimidyletherwie Pyrithiobacsäure, Pyrithiobacsodium, KIH-2023, KIH-6127;
- Sulfonamide wie Flumetsulam, Metosulam;
- Triazolcarboxamide wie Triazofenamid;
- Uracile wie Bromacil, Lenacil, Terbacil;
- ferner Benazolin, Benfuresate, Bensulide, Benzofluor, Bentazon, Butamifos, Cafenstrole, Chlorthaldimethyl, Cinmethylin, Dichlobenil, Endothall, Fluorbentranil, Mefluidide, Perfluidone, Piperophos, Topramezone und Prohexandion-Calcium;
- Sulfonylharnstoffe wie Amidosulfuron, Azimsulfuron, Bensulfuronmethyl, Chlorimuronethyl, Chlorsulfuron, Cinosulfuron, Cyclosulfamuron, Ethametsulfuronmethyl, Flazasulfuron, Halosulfuronmethyl, Imazosulfuron, Metsulfuronmethyl, Nicosulfuron, Primisulfuron, Prosulfuron, Pyrazosulfuronethyl, Rimsulfuron, Sulfometuronmethyl, Thifensulfuronmethyl, Triasulfuron, Tribenuronmethyl, Triflusulfuronmethyl, Tritosulfuron;
- Pflanzenschutz-Wirkstoffe vom Cyclohexenon-Typ wie Alloxydim, Clethodim, Cloproxydim, Cycloxydim, Sethoxydim und Tralkoxydim. Ganz besonders bevorzugte herbizide Wirkstoffe vom Cyclohexenon-Typ sind: Tepraloxydim (vgl. AGROW, Nr. 243, 3.11.95, Seite 21, Caloxydim) und 2-(1-[2-{4-Chlorphenoxy}propyloxylmino]butyl)-3-hydroxy-5-(2H-tetrahydrothiopyran-3-yl)-2-cyclohexen-1-on und vom Sulfonylharnstoff-Typ: N-(((4-methoxy-6-[trifluormethyl]-1,3,5-triazin-2-yl)amino)carbonyl)-2-(trifluormethyl)benzolsulfonamid.

Beispiele für Insektizide, die als erfindungsgemäße wässrige Wirkstoffzusammensetzung formuliert werden können, umfassen:
- Organophosphate wie Acephate, Azinphos-methyl, Chlorpyrifos, Chlorfenvinphos, Diazinon, Dichlorvos, dimethylvinphos, dioxabenzofos, Dicrotophos, Dimethoate, Disulfoton, Ethion, EPN, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Primiphos-ethyl, Pyraclofos, Pyridaphenthion, Sulprophos, Triazophos, Trichlorfon; Tetrachlorvinphos, Vamidothion

- Carbamate wie Alanycarb, Benfuracarb, Bendiocarb, Carbaryl, carbofuran, Carbosulfan, Fenoxycarb, Furathiocarb, Indoxacarb, Methiocarb, Methomyl, Oxamyl, Pirimicarb, Propoxur, Thiodicarb, Triazamate;
- Pyrethroide wie Bifenthrin, Cyfluthrin, Cycloprothrin, Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, alpha-Cypermethrin, Zeta-Cypermethrin, Permethrin;
- Arthropode Wachstumsregulatoren: a) Chitinsyntheseinhibitoren z.B. Benzoylharnstoffe wie Chlorfluazuron, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazole, Clofentazine; b) Ecdysone Antagonisten wie Halofenozide, Methoxyfenozide, Tebufenozide; c) Juvenoide wie Pyriproxyfen, Methoprene, Fenoxycarb; d) Lipid-Biosyntheseinhibitoren wie Spirodiclofen;
- Neonicotinoide wie Flonicamid, Clothianidin, Dinotefuran, Imidacloprid, Thiamethoxam, Nitenpyram, Nithiazin, Acetamiprid, Thiacloprid;
- Weitere unklassifizierte Insektizide wie Abamectin, Acequinocyl, acetamiprid, Amitraz, Azadirachtin, Bensultap Bifenazate, Cartap, Chlorfenapyr, Chlordimeform, Cyromazine, Diafenthiuron, Dinetofuran, Diofenolan, Emamectin, Endosulfan, Ethiprole, Fenazaquin, Fipronil, Formetanate, Formetanate hydrochlorid, gamma-HCH Hydramethylnon, Imidacloprid, Indoxacarb, Isoprocarb, Metolcarb, Pyridaben, Pymetrozine, Spinosad, Tebufenpyrad, Thiamethoxam, Thiocyclam, XMC und Xylylcarb.
- N-Phenylsemicarbazone, wie sie in EP-A 462 456 durch die allgemeine Formel I beschrieben werden, insbesondere Verbindungen der allgemeinen Formel (A) 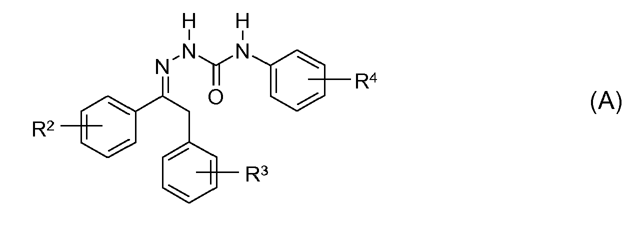 worin R² und R³ unabhängig voneinander für Wasserstoff, Halogen, CN, C₁-C₄-Alkyl, C₁-C₄-Alkoxy, C₁-C₄-Haloalkyl oder C₁-C₄-Haloalkoxy stehen und R⁴ für C₁-C₄-Alkoxy, C₁-C₄-Haloalkyl oder C₁-C₄-Haloalkoxy steht, z. B. Verbindung IV, worin R² für 3-CF₃ und R³ für 4-CN stehen und R⁴ 4-OCF₃ bedeutet.

Brauchbare Wachstumsregulatoren sind z. B. Chlormequatchlorid, Mepiquatchlorid, Prohexadion-Calcium oder die die Gruppe der Gibberelline. Dazu gehören z. B. die Gibberelline GA₁, GA₃, GA₄, GA₅ und GA₇ etc. und die entsprechenden exo-16,17-Dihydrogibberelline sowie die Derivate davon, z. B. die Ester mit C₁-C₄-Carbonsäuren. Erfindungsgemäß bevorzugt ist das exo-16,17-Dihydro-GA₅-13-acetat.

Eine bevorzugte Ausführungsform der Erfindung betrifft die Verwendung der erfindungsgemäßen hyperverzweigten Polymerzusammensetzungen zur Herstellung wässriger Wirkstoffzusammensetzungen von Fungiziden, insbesondere Strobilurinen, Azolen und 6-Aryltriazolo[1,5a]pyrimidinen, wie sie z. B. in WO 98/46608, WO 99/41255 oder WO 03/004465 jeweils durch die allgemeine Formel I beschrieben werden, insbesondere für Wirkstoffe der allgemeinen Formel (B), 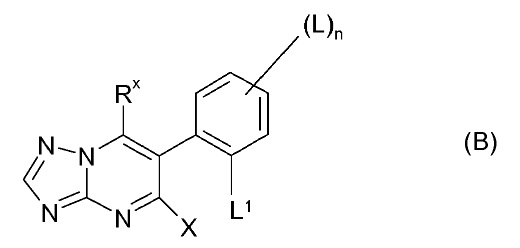 worin:
- R^{x}: für eine Gruppe NR⁵R⁶ steht, oder lineares oder verzweigtes C₁-C₈-Alkyl, das gegebenenfalls durch Halogen, OH, C₁-C₄-Alkoxy, Phenyl oder C₃-C₆-Cycloalkyl substituiert ist, C₂-C₆-Alkenyl, C₃-C₆-Cycloalkyl, C₃-C₆-Cycloalkenyl, Phenyl oder Naphthyl, wobei die 4 zuletzt genannten Reste 1, 2, 3 oder 4 Substituenten aus- gewählt unter Halogen, OH, C₁-C₄-Alkyl, C₁-C₄-Halogenalkoxy, C₁-C₄-Alkoxy und C₁-C₄-Halogenalkyl aufweisen können;
R⁵, R⁶ unabhängig voneinander für Wasserstoff, C₁-C₈-Alkyl, C₁-C₈-Halogenalkyl, C₃-C₁₀-Cycloalkyl, C₃-C₆-Halogencycloalkyl, C₂-C₈-Alkenyl, C₄-C₁₀-Alkadienyl, C₂-C₈-Halogenalkenyl, C₃-C₆-Cycloalkenyl, C₂-C₈-Halogencycloalkenyl, C₂-C₈-Alkinyl, C₂-C₈-Halogenalkinyl oder C₃-C₆-Cycloalkinyl stehen, oder
R⁵ und R⁶ zusammen mit dem Stickstoffatom, an das sie gebunden sind, fünf- bis achtgliedriges Heterocyclyl, welches über N gebunden ist und ein, zwei oder drei weitere Heteroatome aus der Gruppe O, N und S als Ring- glied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogen- alkenyl, C₁-C₆-Alkoxy, C₁-C₆-Halogenalkoxy, C₃-C₆-Alkenyloxy, C₃-C₆-Halo- genalkenyloxy, (exo)-C₁-C₆-Alkylen und Oxy-C₁-C₃-alkylenoxy tragen kann;
- L: ausgewählt ist unter Halogen, Cyano, C₁-C₆-Alkyl, C₁-C₄-Halgoenalkyl, C₁-C₆-Alkoxy, C₁-C₄-Halgoenalkoxy und C₁-C₆-Alkoxycarbonyl;
- L¹: Halogen, C₁-C₆-Alkyl oder C₁-C₆-Halogenalkyl und insbesondere Fluor oder Chlor bedeutet;
- X: für Halogen, C₁-C₄-Alkyl, Cyano, C₁-C₄-Alkoxy oder C₁-C₄-Halogenalkyl und vor- zugsweise für Halogen oder Methyl steht und insbesondere Chlor bedeutet.

### Beispiele für Verbindungen der Formel B sind

5-Chlor-7-(4-methylpiperidin-1-yl)-6-(2,4,6-trifluorphenyl)-[1,2,4]triazolo[1,5-a]pyrimidin,
5-Chlor-7-(4-methylpiperazin-1-yl)-6-(2,4,6-trifluorphenyl)-[1,2,4]triazolo[1,5-a]pyrimidin,
5-Chlor-7-(morpholin-1-yl)-6-(2,4,6-trifluorphenyl)-[1,2,4]triazolo[1,5-a]pyrimidin,
5-Chlor-7-(piperidin-1-yl)-6-(2,4,6-trifluorphenyl)-[1,2,4]triazolo[1,5-a]pyrimidin,
5-Chlor-7-(morpholin-1-yl)-6-(2,4,6-trifluorphenyl)-[1,2,4]triazolo[1,5-a]pyrimidin,
5-Chlor-7-(isopropylamino)-6-(2,4,6-trifluorphenyl)-[1,2,4]triazolo[1,5-a]pyrimidin,
5-Chlor-7-(cyclopentylamino)-6-(2,4,6-trifluorphenyl)-[1,2,4]triazolo[1,5-a]pyrimidin,
5-Chlor-7-(2,2,2-trifluorethylamino)-6-(2,4,6-trifluorphenyl)-[1,2,4]triazolo[1,5-a]-pyrimidin,
5-Ch lor-7-(1,1,1-trifluorpropan-2-ylamino)-6-(2,4,6-trifluorphenyl)-[1,2,4]triazolo[1,5-a]-pyrimidin,
5-Chlor-7-(3,3-dimethylbutan-2-ylamino)-6-(2,4,6-trifluorphenyl)-[1,2,4]triazolo[1,5-a]-pyrimidin,
5-Chlor-7-(cyclohexylmethyl)-6-(2,4,6-trifluorphenyl)-[1,2,4]triazolo[1,5-a]pyrimidin,
5-Chlor-7-(cyclohexyl)-6-(2,4,6-trifluorphenyl)-[1,2,4]triazolo[1,5-a]pyrimidin,
5-Chlor-7-(2-methylbutan-3-yl)-6-(2,4,6-trifluorphenyl)-[1,2,4]triazolo[1,5-a]pyrimidin,
5-Chlor-7-(3-methylpropan-1-yl)-6-(2,4,6-trifluorphenyl)-[1,2,4]triazolo[1,5-a]pyrimidin,
5-Ch lor-7-(4-methylcyclohexan-1-yl)-6-(2,4,6-trifluorphenyl)-[1,2,4]triazolo[1,5-a]-pyrimidin,
5-Chlor-7-(hexan-3-yl)-6-(2,4,6-trifluorphenyl)-[1,2,4]triazolo[1,5-a]pyrimidin,
5-Chlor-7-(2-methylbutan-1-yl)-6-(2,4,6-trifluorphenyl)-[1,2,4]triazolo[1,5-a]pyrimidin,
5-Chlor-7-(3-methylbutan-1-yl)-6-(2,4,6-trifluorphenyl)-[1,2,4]triazolo[1,5-a]pyrimidin,
5-Chlor-7-(1-methylpropan-1-yl)-6-(2,4,6-trifluorphenyl)-[1,2,4]triazolo[1,5-a]pyrimidin,
5-Methyl-7-(4-methylpiperidin-1 -yl)-6-(2,4,6-trifluorphenyl)-[1,2,4]triazolo[1,5-a]-pyrimidin,
5-Methyl-7-(4-methylpiperazin-1-yl)-6-(2,4,6-trifluorphenyl)-[1,2,4]triazolo[1,5-a]-pyrimidin,
5-Methyl-7-(morpholin-1-yl)-6-(2,4,6-trifluorphenyl)-[1,2,4]triazolo[1,5-a]pyrimidin,
5-Methyl-7-(piperidin-1-yl)-6-(2,4,6-trifluorphenyl)-[1,2,4]triazolo[1,5-a]pyrimidin,
5-Methyl-7-(morpholin-1-yl)-6-(2,4,6-trifluorphenyl)-[1,2,4]triazolo[1,5-a]pyrimidin,
5-Methyl-7-(isopropylamino)-6-(2,4,6-trifluorphenyl)-[1,2,4]triazolo[1,5-a]pyrimidin,
5-Methyl-7-(cyclopentylamino)-6-(2,4,6-trifluorphenyl)-[1,2,4]triazolo[1,5-a]pyrimidin,
5-Methyl-7-(2,2,2-trifluorethylamino)-6-(2,4,6-trifluorphenyl)-[1,2,4]triazolo[1,5-a]-pyrimidin,
5-Methyl-7-(1,1,11-trifluorpropan-2-ylamino)-6-(2,4,6-trifluorphenyl)-[1,2,4]triazolo[1,5-a]-pyrimidin,
5-Methyl-7-(3,3-dimethylbutan-2-ylamino)-6-(2,4,6-trifluorphenyl)-[1,2,4]triazolo[1,5-a]-pyrimidin,
5-Methyl-7-(cyclohexylmethyl)-6-(2,4,6-trifluorphenyl)-[1,2,4]triazolo[1,5-a]pyrimidin,
5-Methyl-7-(cyclohexyl)-6-(2,4,6-trifluorphenyl)-[1,2,4]triazolo[1,5-a]pyrimidin,
5-Methyl-7-(2-methylbutan-3-yl)-6-(2,4,6-trifluorphenyl)-[1,2,4]triazolo[1,5-a]pyrimidin,
5-Methyl-7-(3-methylpropan-1-yl)-6-(2,4,6-trifluorphenyl)-[1,2,4]triazolo[1,5-a]pyrimidin,
5-Methyl-7-(4-methylcyclohexan-1-yl)-6-(2,4,6-trifluorphenyl)-[1,2,4]triazolo[1,5-a]-pyrimidin,
5-Methyl-7-(hexan-3-yl)-6-(2,4,6-trifluorphenyl)-[1,2,4]triazolo[1,5-a]pyrimidin,
5-Methyl-7-(2-methylbutan-1-yl)-6-(2,4,6-trifluorphenyl)-[1,2,4]triazolo[1,5-a]pyrimidin,
5-Methyl-7-(3-methylbutan-1-yl)-6-(2,4,6-trifluorphenyl)-[1,2,4]triazolo[1,5-a]pyrimidin und 5-Methyl-7-(1-methylpropan-1-yl)-6-(2,4,6-trifluorphenyl)-[1,2,4]triazolo[1,5-a]-pyrimidin.

Eine weitere bevorzugte Ausführungsform der Erfindung betrifft die Verwendung stickstoffatomhaltiger hyperverzweigter Polymere zur Herstellung wässriger Wirkstoffzusammensetzungen von Insektiziden, insbesondere von Arylpyrrolen wie Chlorfenapyr, von Pyrethroiden wie Bifenthrin, Cyfluthrin, Cycloprothrin, Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, alpha-Cypermethrin, Zeta-Cypermethrin und Permethrin, von Neonicotinoiden und von Semicarbazonen der Formel A.

Weiterhin können die erfindungsgemäß zu verwendenden stickstoffatomhaltigen, hyperverzweigten Polymere als Solubilisatoren für in Wasser schwerlösliche oder unlösliche UV-Absorber verwendet werden.

Der Betriff UV-Absorber umfasst im Rahmen der vorliegenden Erfindung UV-A-, UV-Bund/oder Breitbandfilter.

Vorteilhafte Breitbandfilter, UV-A- oder UV-B-Filtersubstanzen sind beispielsweise Vertreter der folgenden Verbindungsklassen:

Bis-Resorcinyltriazinderivate mit der folgenden Struktur: 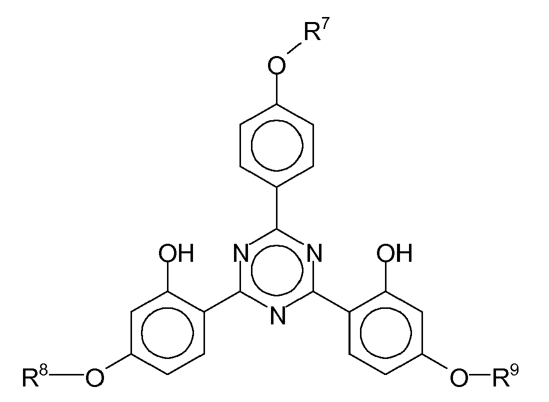 wobei R⁷, R⁸ und R⁹ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb^{®} S bei der CIBA-Chemikalien GmbH erhältlich ist.

Auch andere UV-Filtersubstanzen, welche das Strukturmotiv 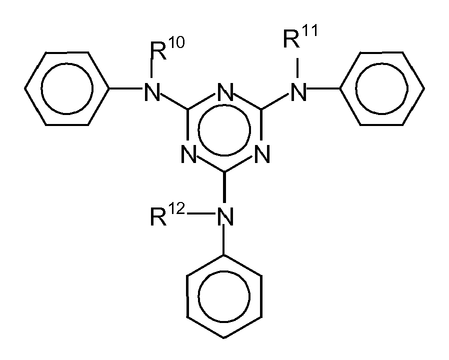 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 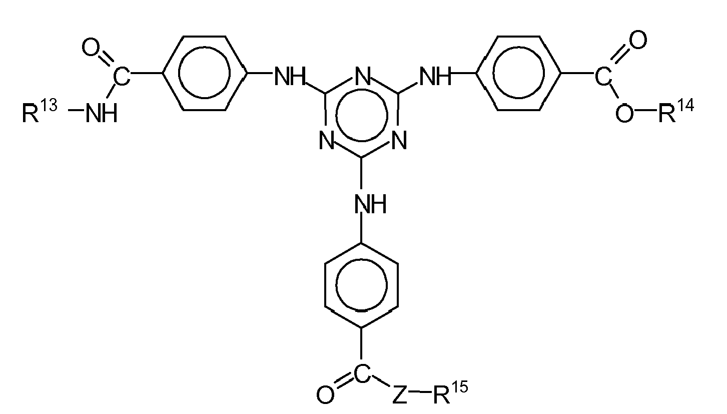 wiedergegeben wird, wobei
- R¹³: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- Z ein: Sauerstoffatom oder eine NH-Gruppe darstellt,
- R¹⁴: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 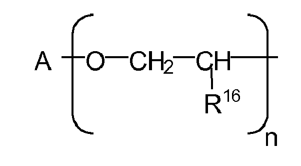 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit ei- ner oder mehreren C₁-C₄- Alkylgruppen,
- R¹⁶: ein Wasserstoffatom oder eine Methylgruppe darstellt,
- n: eine Zahl von 1 bis 10 darstellt,
- R¹⁵: einen verzweigten oder unverzweigten C1-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 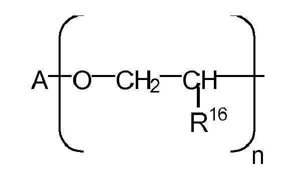 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit ei- ner oder mehreren C₁-C₄-Alkylgruppen,
- R¹⁶: ein Wasserstoffatom oder eine Methylgruppe darstellt,
- n: eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 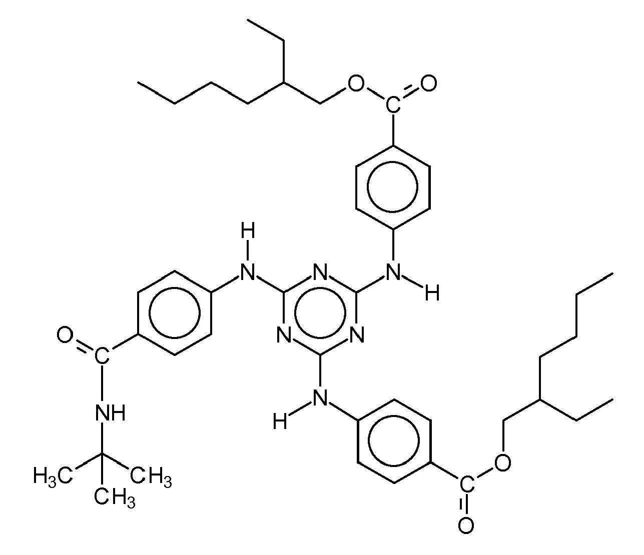 wiedergegeben wird, welches im Folgenden auch als Dioctylbutylamidotriazon (INCI: Diethylhexylbutamidotriazone) bezeichnet wird und unter der Handelsbezeichnung UVASORB^{®} HEB bei Sigma 3V erhältlich ist.

Vorteilhaft im Sinne der vorliegenden Erfindung ist auch ein symmetrisch substituiertes s-Triazin, das 2,4,6-Trianilino-p-(carbo-2'-ethylhexyl-1'-oxy)-1,3,5-triazin(INCI: Ethylhexyl Triazone), welches von der BASF Aktiengesellschaft unter der Warenbezeichnung UVINUL^{®} T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 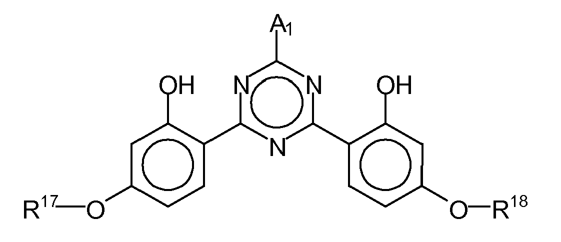 wiedergegeben wird, wobei R¹⁷ und R¹⁸ u. a. C₃-C₁₈-Alkyl oder C₂-C₁₈-Alkenyl und A₁ einen aromatischen Rest repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-propyloxy)-2-hydroxypropyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Vorteilhafte öllösliche UV-B- und/oder Breitband-Filtersubstanzen sind z. B.:
3-Benzylidencampher-Derivate, vorzugsweise 3-(4-Methylbenzyliden)campher, 3-Benzylidencampher;
4-Aminobenzoesäure-Derivate, vorzugsweise
4-(Dimethylamino)-benzoesäure(2-ethylhexyl)ester,
4-(Dimethylamino)benzoesäureamylester,

Derivate des Benzophenons, vorzugsweise 2-Hydroxy-4-methoxybenzophenon (unter der Handelsbezeichnung Uvinul^{®} M40 von der Fa. BASF erhältlich) 2-Hydroxy-4-methoxy-4'-methylbenzophenon, 2,2'-Dihydroxy-4-methoxybenzophenon, 2,2',4,4'-Tetrahydroxybenzophenon (unter der Handelsbezeichnung Uvinul^{®} D 50 von der Fa. BASF erhältlich).

Besonders vorteilhafte bei Raumtemperatur flüssige UV-Filtersubstanzen im Sinne der vorliegenden Erfindung sind Homomenthylsalicylat, 2-Ethylhexyl-2-cyano-3,3-diphenylacrylat, 2-Ethylhexyl-2-hydroxybenzoat und Ester der Zimtsäure, vorzugsweise 4-Methoxyzimtsäure(2-ethylhexyl)ester und 4-Methoxyzimtsäureisopentylester.

Homomethylsalicylat (INCI: Homosalate) zeichnet sich durch die folgende Struktur aus:

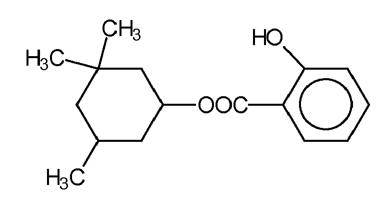

2-Ethylhexyl-2-cyano-3,3-diphenylacrylat (INCI: Octocrylene) ist von BASF unter der Bezeichnung Uvinul^{®} N 539T erhältlich und zeichnet sich durch folgende Struktur aus:

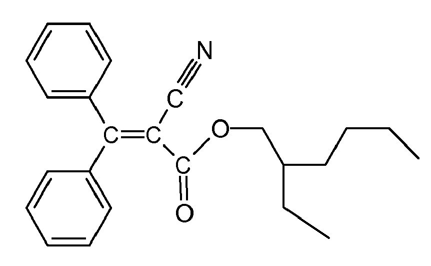

2-Ethylhexyl-2-hydroxybenzoat (2-Ethylhexylsalicylat, Octylsalicylat, INCI: Ethylhexyl Salicylate) ist beispielsweise bei Haarmann & Reimer unter der Handelsbezeichnung Neo Heliopan^{®} OS erhältlich und zeichnet sich durch die folgende Struktur aus:

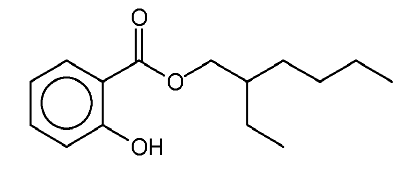

4-Methoxyzimtsäure(2-ethylhexyl)ester (2-Ethylhexyl-4-methoxycinnamat, INCI: Ethylhexyl Methoxycinnamate) ist beispielsweise bei Fa. BASF unter der Handelsbezeichnung Uvinul^{®} MC 80 erhältlich und zeichnet sich durch die folgende Struktur aus:

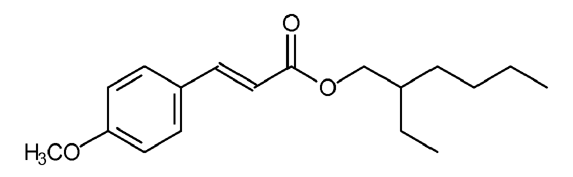

4-Methoxyzimtsäureisopentylester (Isopentyl-4-methoxycinnamat, INCI: Isoamyl p-Methoxycinnamate) ist beispielsweise bei Haarmann & Reimer unter der Handelsbezeichnung Neo Heliopan^{®} E 1000 erhältlich und zeichnet sich durch die folgende Struktur aus:

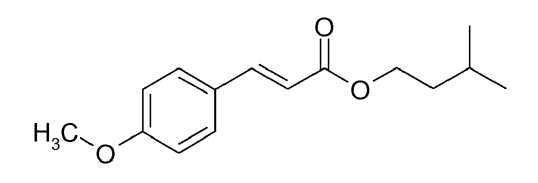

Vorteilhafte Dibenzoylmethanderivate im Sinne der vorliegenden Erfindung sind, insbesondere das 4-(tert.-Butyl)-4'-methoxydibenzoylmethan (CAS-Nr. 70356-09-1), welches von BASF unter der Marke Uvinul^{®} BMBM und von Merck unter der Handelsbezeichnung Eusolex^{®} 9020 verkauft wird zeichnet sich durch folgende Struktur aus:

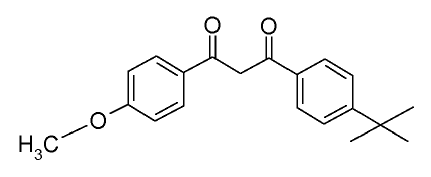

Ein weiteres vorteilhaftes Dibenzoylmethanderivat ist das 4-Isopropyl-Dibenzoylmethan (CAS-Nr. 63250-25-9), welches von Merck unter dem Namen Eusolex^{®} 8020 verkauft wird. Das Eusolex 8020 zeichnet sich durch folgende Struktur aus:

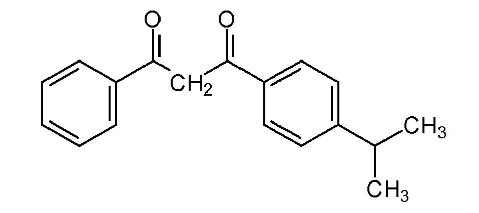

Benzotriazole zeichnen sich durch die folgende Strukturformel aus: 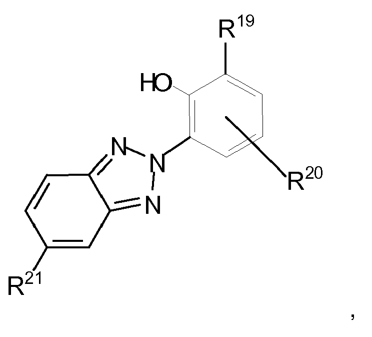 worin

R¹⁹ und R²⁰ unabhängig voneinander lineare oder verzweigte, gesättigte oder ungesättigte, substituierte (z. B. mit einem Phenylrest substituierte) oder unsubstituierte Alkylreste mit 1 bis 18 Kohlenstoffatomen stehen.

Vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches von Fa. Chimex unter der Marke Mexoryl^{®} XL verkauft wird und durch die folgende chemische Strukturformel 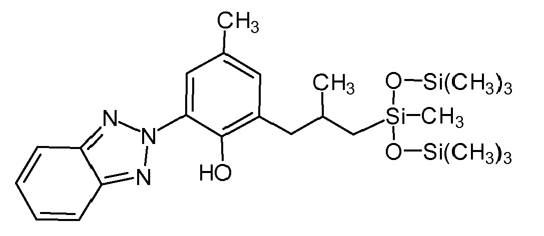 gekennzeichnet ist.

Weitere vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-n-octoxy-5'-benzoyl]diphenylmethan, 2,2' Methylen-bis-[6-(2H-benzotriazol-2-yl)-4-(methyl)phenol], 2,2'-Methylen-bis-[6-(2H-benzotriazol-2-yl)-4-(1,1,3,3-tetramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Ein weiterer im Sinne der vorliegenden Erfindung vorteilhafter UV-Filter ist die in EP-A-0 916 335 beschriebene Diphenylbutadienverbindung der folgenden Formel.

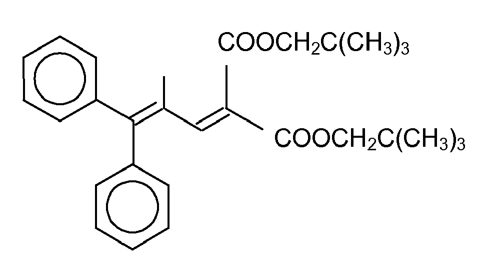

Ein weiterer im Sinne der vorliegenden Erfindung vorteilhafter UV-A-Filter ist der in EP-A-0 895 776 beschriebene 2-(4-Ethoxy-anilinomethylen)-propandicarbonsäurediethylester der folgenden Formel.

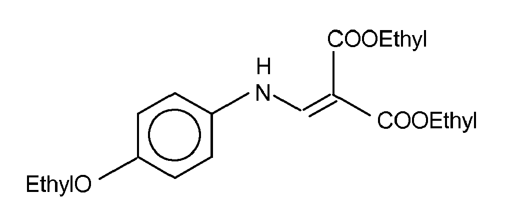

Vorteilhaft im Sinne der vorliegenden Erfindung ist ebenfalls ein aminosubstituiertes Hydroxybenzophenon der folgenden Formel: 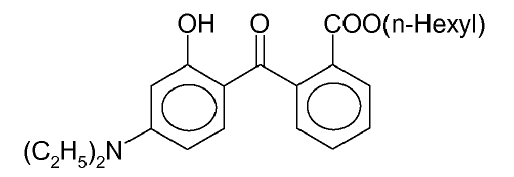 welche von der BASF Aktiengesellschaft als UV-A Filter unter der Warenbezeichnung UVINUL^{®} A Plus vertrieben wird.

Die erfindungsgemäß eingesetzten stickstoffhaltigen hyperverzweigten Polymere eignen sich auch vorteilhaft als Solubilisatoren für kosmetische Zusammensetzungen. Gegenstand der Erfindung ist daher auch ein kosmetisches Mittel, enthaltend
A) wenigstens ein stickstoffatomhaltiges, hyperverzweigtes Polymer, wie zuvor definiert,
B) wenigstens einen kosmetisch akzeptablen Wirk- oder Effektstoff, der in Wasser bei 25 °C und 1013 mbar eine Löslichkeit unterhalb 10 g/l aufweist, und
C) gegebenenfalls wenigstens einen weiteren, von B) verschiedenen kosmetisch akzeptablen Wirk- oder Hilfsstoff.

Vorzugsweise sind die Komponenten B) und C) nach Maßgabe ihrer Löslichkeit ausgewählt unter kosmetisch akzeptablen Trägern, Emulgatoren, Tensiden, Konservierungsmitteln, Parfümölen, Verdickern, Haarpolymeren, Haar- und Hautconditionern, wasserlöslichen oder dispergierbaren siliconhaltigen Polymeren, Bleichmitteln, Gelbildnern, Pflegemitteln, Färbemitteln, Tönungsmitteln, Bräunungsmitteln, Farbstoffen, Pigmenten, Antischuppenmitteln, Lichtschutzmitteln, desodorierenden Wirkstoffen, Vitaminen, Pflanzenextrakten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

Eine umfassende Darstellung kosmetischer Hilfsstoffe findet sich in H. P. Fiedler, Lexikon der Hilfsstoffe für Pharmazie, Kosmetik und angrenzende Gebiete, 4. Auflage, Aulendorff: ECV-Editio-Cantor-Verlag, 1996. Eine umfassende Darstellung kosmetischer Grund-, Hilfs- und Wirkstoffe sowie geeignete Formulierungen finden sich weiterhin in K. Schrader, Grundlagen und Rezepturen der Kosmetika, 2. Auflage, Hüthig-Verlag Heidelberg (1989).

Geeignete nur gering wasserlösliche oder wasserunlösliche kosmetisch akzeptable Träger B) sind z. B. ausgewählt unter Ölen, Fetten, Wachsen, gesättigten acyclischen und cyclischen Kohlenwasserstoffen, Fettsäuren, Fettalkoholen, etc. und Mischungen davon.

Geeignete wässrige Träger C) sind z. B. ausgewählt unter Wasser, wassermischbaren organischen Lösungsmitteln, vorzugsweise C₁-C₄-Alkanolen, und Mischungen davon.

Bei den erfindungsgemäßen kosmetischen Mitteln handelt es sich um Solubilisate auf Wasser oder Wasser/Alkohol-Basis. Die erfindungsgemäß zu verwendenden Solubilisatoren A) werden vorzugsweise im Verhältnis von 0,2:1 bis 20:1, bevorzugt 1:1 bis 15:1, besonders bevorzugt 2:1 bis 12:1 zum schwerlöslichen kosmetischen Wirk- oder Effektstoff B) eingesetzt.

Der Gehalt an erfindungsgemäß zu verwendendem Solubilisator A) in den kosmetischen Mitteln liegt vorzugsweise im Bereich von 0,01 bis 50 Gew.-%, bevorzugt 0,1 bis 40 Gew.-%, besonders bevorzugt 1 bis 30 Gew.%, bezogen auf das Gesamtgewicht des Mittels.

Die erfindungsgemäßen kosmetischen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Rizinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete hydrophile Träger C) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc.

Bei den erfindungsgemäßen kosmetischen Mitteln kann es sich z. B. um hautkosmetische, dermatologische oder haarkosmetische Mittel handeln.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens einen schwerlöslichen UV-Absorber, wie zuvor definiert.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-Bund/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon.

Geeignet sind weiterhin Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Zusätzlich können den kosmetischen Mitteln weitere Hilfsstoffe zugesetzt werden, beispielsweise nichtionische, kationische oder anionische Tenside wie Alkylpolyglycoside, Fettalkoholsulfate, Fettalkoholethersulfate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolventien wie Ethylenglykol, Propylenglykol, Glycerin u. a.

Als weitere Bestandteile können natürliche oder synthetische Verbindungen, z. B. Lanolinderivate, Cholesterinderivate, Isopropylmyristat, Isopropylpalmitat, Elektrolyte, Farbstoffe, Konservierungsmittel, Säuren (z. B. Milchsäure, Zitronensäure) zugesetzt werden.

Geeignete kosmetische Mittel sind beispielsweise Badezusatzpräparate wie Badeöle, Rasierwässer, Gesichtswässer, Mundwässer, Haarwässer, Eau de Cologne, Eau de Toilette sowie Sonnenschutzmittel.

Bei der Herstellung der Solubilisate für kosmetische Formulierungen können die erfindungsgemäß zu verwendenden Copolymere als 100 %ige Substanz oder bevorzugt als wässrige Lösung eingesetzt werden.

Üblicherweise wird der Solubilisator in Wasser gelöst und mit dem jeweils zu verwendenden schwerlöslichen kosmetischen Wirkstoff intensiv vermischt.

Es kann aber auch der Solubilisator mit dem jeweils zu verwendenden schwerlöslichen kosmetischen Wirkstoff intensiv vermischt werden und anschließend unter ständigem Rühren mit demineralisiertem Wasser versetzt werden.

Ein weiterer Gegenstand der Erfindung ist daher ein pharmazeutisches Mittel, enthaltend
A) wenigstens ein stickstoffatomhaltiges hyperverzweigtes Polymer, wie zuvor definiert,
B) wenigstens einen pharmazeutisch akzeptablen Wirkstoff, der in Wasser bei 25 °C und 1013 mbar eine Löslichkeit unterhalb 10 g/l aufweist, und
C) gegebenenfalls wenigstens einen weiteren, von B) verschiedenen pharmazeutisch akzeptablen Wirk- oder Hilfsstoff.

Die erfindungsgemäß zu verwendenden Copolymerisate eignen sich ebenso für die Verwendung als Solubilisator in pharmazeutischen Zubereitungen jeder Art.

Die Formulierungsgrundlage der erfindungsgemäßen pharmazeutischen Mitteln enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden. Auch für diesen Zweck eignen sich die erfindungsgemäßen Copolymere um einen schwerlöslichen Arzneistoff zu verarbeiten.

Pharmazeutische Formulierungen der oben genannten Art können durch Verarbeiten der erfindungsgemäß zu verwendenden Copolymere mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Besonders bevorzugt sind von den oben genannten pharmazeutischen Mitteln solche, bei denen es sich um parenteral applizierbare Formulierungen handelt.

Der Gehalt an erfindungsgemäßem Solubilisator in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Ein weiterer Aspekt der vorliegenden Erfindung betrifft die Verwendung der genannten Copolymere als Solubilisatoren in molekulardispersen Systemen. Feststoffdispersionen, also homogene feinstdisperse Phasen von zwei oder mehreren Feststoffen sowie ihr Sonderfall der so genannten "festen Lösungen" (molekulardisperse Systeme), sowie ihr Einsatz in der pharmazeutischen Technologie sind allgemein bekannt (vgl. Chiou und Riegelmann, J. Pharm. Sci., 1971, 60, 1281 - 1300. Daneben betrifft die vorliegende Erfindung auch feste Lösungen die mindestens ein erfindungsgemäß zu verwendendes Copolymer enthalten.

Die Herstellung von festen Lösungen kann mit Hilfe von Schmelzeverfahren oder nach dem Lösungsverfahren erfolgen.

Als polymerer Hilfsstoff, d.h. Solubilisator für die Herstellung solcher Feststoffdispersionen bzw. fester Lösungen eignen sich die erfindungsgemäßen Copolymere.

Nach dem Schmelzeverfahren können beispielsweise ein schwerlöslicher Wirkstoff B) und das gewählte Copolymer A) im gewünschten Verhältnis, z. B. zu gleichen Teilen abgewogen und gemischt werden. Zur Mischung eignet sich beispielsweise ein Freifallmischer. Die Mischung kann anschließend, z. B. in einem Zweischneckenextruder, extrudiert werden. Der Durchmesser des so erhaltenen, abgekühlten Produktstrangs, bestehend aus einer festen Lösung des gewählten Wirkstoffes in dem gewählten erfindungsgemäß zu verwendenden Copolymeren, ist abhängig vom Durchmesser der Perforation der Lochscheiben des Extruders. Durch das Abschneiden der gekühlten Produktstränge mit Hilfe eines rotierenden Messers können zylindrische Partikel gewonnen werden, deren Höhe abhängig ist vom Abstand zwischen Lochscheibe und Messer. Der mittlere Durchmesser der zylindrischen Partikel beträgt in der Regel etwa 1000 bis etwa 3000 µm, die Höhe in der Regel etwa 2000 bis etwa 5000 µm. Größere Extrudate können in einem nachgeschalteten Schritt zerkleinert werden.

Alternativ kann man feste Lösung auch im Lösungsverfahren herstellen. Hierzu löst man üblicherweise den gewählten schwerlöslichen Wirkstoff B) und das gewählte, als Solubilisator dienende erfindungsgemäß zu verwendende Copolymer A) in einem geeigneten Lösungsmittel. Anschließend wird die Lösung üblicherweise in eine geeignete Form gegossen, und das Lösungsmittel, beispielsweise durch Trocknung, entfernt. Die Trocknungsbedingungen wählt man vorteilhaft je nach den Eigenschaften von Wirkstoff (z. B Thermolabilität) und Lösungsmittel (z. B. Siedepunkt).

Unter Beachtung des Materialverhaltens kann der entstandene Formling bzw. das Extrudat beispielsweise mit einer geeigneten Mühle (z. B. Stiftmühle) zerkleinert werden. Die feste Lösung zerkleinert man vorteilhafterweise bis zu einer mittleren Teilchengröße von weniger als etwa 2000 µm, bevorzugt weniger als etwa 1000 µm und besonders bevorzugt weniger als etwa 500 µm.

Mit geeigneten Hilfsstoffen kann nun das entstandene Schüttgut zu einer Tablettiermischung oder zu einem Kapselfüllgut verarbeitet werden. Die Tablettierung führt man vorteilhaft so durch, dass man Tabletten mit Härte von größer etwa 35 N, bevorzugt größer etwa 60 N, besonders bevorzugt von etwa 80 bis etwa 100 N erhält.

Die so erhältlichen Formulierungen können wie herkömmliche Formulierungen erforderlichenfalls mit geeigneten Überzugsmaterialien zur Erzielung von Magensaftresistenz, Retardierung, Geschmacksmaskierung usw. überzogen werden.

Neben der Anwendung in der Kosmetik und Pharmazie eignen sich die erfindungsgemäß zu verwendenden Copolymere auch als Solubilisatoren im Lebensmittelbereich für schwer wasserlösliche oder wasserunlösliche Nähr-, Hilfs- oder Zusatzstoffe, wie z. B. fettlösliche Vitamine oder Carotinoide. Als Beispiele seien klare, mit Carotinoiden gefärbte Getränke genannt. Gegenstand der Erfindung sind daher auch lebensmitteltechnische Zubereitungen, die mindestens eines der erfindungsgemäß zu verwendenden Copolymere als Solubilisator enthalten. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die genannten Copolymere auch als Solubilisatoren für Futtermittelzusätze für die Tierernährung.

Die Anwendung der erfindungsgemäß zu verwendenden Copolymere als Solubilisatoren in der Agrochemie kann u.a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

Außerdem eignen sich die erfindungsgemäß eingesetzten stickstoffatomhaltigen hyperverzweigten Polymere zur Herstellung wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

Beispiele für Effektstoffe, die als erfindungsgemäße wässrige Wirkstoffzusammensetzung formuliert werden können, sind:
Farbstoffe: z. B. die in DE-A 10245209 beschriebenen Farbstoffe sowie die gemäß Colour-Index als Disperse-Farbstoffe und als Solvent-Farbstoffe bezeichneten Verbindungen, die auch als Dispersionsfarbstoffe bezeichnet werden. Eine Zusammenstellung geeigneter Dispersionsfarbstoffe findet sich beispielsweise in Ullmanns Enzyklopädie der technischen Chemie, 4. Auflage, Bd. 10, S. 155-165 (siehe auch Bd. 7, S. 585ff - Anthrachinonfarbstoffe; Bd. 8, S. 244ff - Azofarbstoffe; Bd. 9, S. 313ff - Chinophthalonfarbstoffe). Auf diese Literaturstelle und die darin genannten Verbindungen wird hiermit ausdrücklich Bezug genommen. Erfindungsgemäß geeignete Dispersionsfarbstoffe und Solvent-Farbstoffe umfassen verschiedenste Farbstoffklassen mit unterschiedlichen Chromophoren, beispielsweise Anthrachinonfarbstoffe, Monoazo- und Disazofarbstoffe, Chinophthalone, Methin- und Azamethinfarbstoffe, Naphthalimidfarbstoffe, Naphthochinonfarbstoffe und Nitrofarbstoffe. Beispiele für erfindungsgemäß geeignete Dispersionsfarbstoffe sind die Dispersionsfarbstoffe der folgenden Colour-Index Liste: C. I. Disperse Yellow 1 - 228, C. I. Disperse Orange 1 - 148, C. I. Disperse Red 1 - 349, C. I. Disperse Violet 1 - 97, C. I. Disperse Blue 1 - 349, C. I. Disperse Green 1 - 9, C. I. Disperse Brown 1 - 21, C. I. Disperse Black 1 - 36. Beispiele für erfindungsgemäß geeignete Solvent-Farbstoffe sind die Verbindungen der folgenden Colour-Index Liste: C. I. Solvent Yellow 2 - 191, C. I. Solvent Orange 1 - 113, C. I. Solvent Red 1 - 248, C. I. Solvent Violet 2 - 61, C. I. Solvent Blue 2 - 143, C. I. Solvent Green 1 - 35, C. I. Solvent Brown 1 - 63, C. I. Solvent Black 3 - 50. Erfindungsgemäß geeignete Farbstoffe sind weiterhin Derivate des Naphthalins, des Anthracens, des Perylens, des Terylens, des Quarterylens, sowie Diketopyrrolopyrrolfarbstoffe, Perinonfarbstoffe, Cumarinfarbstoffe, Isoindolin- und Isoindolinonfarbstoffe, Porphyrinfarbstoffe, Phthalocyanin- und Naphthalocyaninfarbstoffe.

Neben den vorgenannten Bestandteilen können die erfindungsgemäßen Wirk- und Effektstoffzusammensetzungen auch konventionelle oberflächenaktive Substanzen und sonstige Additive enthalten. Zu den oberflächenaktiven Substanzen zählen Tenside, Dispergierhilfsmittel und Netzmittel. Zu den sonstigen Additiven zählen insbesondere Verdickungsmittel, Entschäumer, Konservierungsmittel, Frostschutzmittel Stabilisierungsmittel, etc.

Prinzipiell brauchbar sind anionische, kationische, nichtionische und amphotere Tenside, wobei Polymertenside sowie Tenside mit Heteroatomen in der hydrophoben Gruppe eingeschlossen sind.

Zu den anionischen Tensiden gehören beispielsweise Carboxylate, insbesondere Alkali-, Erdalkali- und Ammoniumsalze von Fettsäuren, z. B. Kaliumstearat, die üblicherweise auch als Seifen bezeichnet werden; Acylglutamate; Sarkosinate, z. B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Alkylbenzolsulfonsäuren, wie beispielsweise Lignin- und Phenolsulfonsäure, Naphthalin-und Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyl und Harnstoff, Mono- oder Dialkylbernsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide.

Zu den nichtionischen Tensiden gehören beispielsweise:
- Fettalkoholpolyoxyethylenester, beispielsweise Laurylalkoholpolyoxyethylenetheracetat,
- Alkyl-Polyoxyethylen- und -polyoxypropylenether, z. B. von iso-Tridecylalkohol und Fettalkohol-Polyoxyethylenether,
- Alkylarylalkohol-Polyoxyethylenether, z. B. Octylphenol-Polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Fettalkoholalkoxylate und Oxoalkoholalkoxylate, insbesondere vom Typ RO-(R₁₈0)ᵣ(R₁₉O)ₛR₂₀ mit R₁₈ und R₁₉ unabhängig voneinander = C₂H₄, C₃H₆, C₄H₈ und R₂₀ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie iso-Tridecylalkohol und Oleylalkoholpolyoxyethylenether,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z. B. Tetradecyldimethylaminoxid.

Weitere Tenside, die hier beispielhaft genannt werden sollen, sind Perfluortenside, Silikontenside, Phospholipide, wie beispielsweise Lecithin oder chemisch modifizierte Lecithine, Aminosäuretenside, z. B. N-Lauroylglutamat.

Sofern nicht spezifiziert, handelt es sich bei den Alkylketten der oben aufgeführten Tenside um lineare oder verzweigte Reste mit üblicherweise 8 bis 20 Kohlenstoffatomen.

In einer Ausführungsform enthalten die erfindungsgemäßen wässrigen Wirkstoffzusammensetzung nicht mehr als 10 Gew.-%, vorzugsweise nicht mehr als 5 Gew.-% und insbesondere nicht mehr als 3 Gew.-%, z. B. 0,01 bis 5 Gew.-% oder 0,1 bis 3 Gew.-% an konventionellen oberflächenaktiven Substanzen, jeweils bezogen auf die Gesamtmenge an Wirkstoff und Polymerzusammensetzung. Die konventionellen oberflächenaktiven Substanzen machen dann vorzugsweise nicht mehr als 5 Gew.-% und insbesondere nicht mehr als 3 Gew.-%, z. B. 0,01 bis 5 Gew.-% oder 0,1 bis 3 Gew.-%, bezogen auf das Gesamtgewicht der Zusammensetzung aus.

Je nach Anwendung kann es jedoch von Vorteil sein, wenn die erfindungsgemäßen Wirkstoffzusammensetzungen mit oberflächenaktiven Substanzen formuliert werden. Dann liegt der Anteil an konventioneller oberflächenaktiver Substanz häufig im Bereich von 0,5 bis 30 Gew.-%, insbesondere im Bereich von 1 bis 20 Gew.-%, bezogen auf die Gesamtmenge an Wirkstoff und Polymerzusammensetzung, bzw. im Bereich von 0,2 bis 20 Gew.-% und insbesondere im Bereich von 0,5 bis 15 Gew.-%, bezogen auf das Gesamtgewicht der formulierten Zusammensetzung.

Auch wenn ein Vorteil der erfindungsgemäßen Zusammensetzungen ihr geringer Gehalt an flüchtigen organischen Substanzen ist, kann es für einige Anwendungen erwünscht sein, die erfindungsgemäßen Zusammensetzungen mit organischen Lösungsmitteln, Ölen und Fetten, vorzugsweise solchen Lösungsmitteln oder Ölen und Fetten, die umweltverträglich oder biokompatibel sind, z. B. die vorgenannten mit Wasser mischbaren Lösungsmittel oder Lösungsmittel, Ölen oder Fetten die mit Wasser nicht oder nur sehr begrenzt mischbar sind, einzusetzen, z. B.:
- Paraffinöle, aromatische Kohlenwasserstoffe und aromatische Kohlenwasserstoffgemische, z. B. Xylole, Solvesso 100, 150 oder 200, und dergleichen,
- Phenole und Alkylphenole, z. B. Phenol, Hydrochinon, Nonylphenol, etc.
- Ketone mit mehr als 4 C-Atomen wie Cyclohexanon, Isophoron, Isopheron, Acetophenon, Acetonaphthon,
- Alkohole mit mehr als 4 C-Atomen wie acetylierter Lanolinalkohol, Cetylalkohol, 1-Decanol, 1-Heptanol, 1-Hexanol, Isooctadecanol, Isopropylalkohol, Oleylalkohol, Benzylalkohol,
- Carbonsäureester, z. B. Adipinsäuredialkylester wie Adipinsäurebis(2-ethylhexyl)ester, Phthalsäuredialkylester wie Phthalsäurebis(2-ethylhexyl)ester, Essigsäurealkylester (auch verzweigte Alkylgruppen) wie Ethylacetat und Acetoessigsäureethylester, Stearate wie Butylstearat, Glycerinmonostearat, Citrate wie Acetyltributylcitrat, weiterhin Cetyloctanoat, Methyloleat, Methyl-p-hydroxyvbenzoat, Methyltetradecanoat, Propylp-hydroxybenzoat, Methylbenzoat, Milchsäureester wir Isopropyllacttat, Butyllactat und 2-Ethylhexyllactat,
- Pflanzenöle wie Palmöl, Rapsöl, Rizinusöl und Derivate davon wie z. B. oxydiert, Kokusnussöl, Lebertran, Maiskeimöl, Sojabohnenöl, Leinsamenöl, Olivenöl, Erdnussöl, Färberdistelöl, Sesamsamenöl, Grapefruitöl, Basilikumöl, Aprikosenöl, Ingweröl, Geranienöl, Orangenöl, Rosmarienöl, Macadamiaöl, Zwiebelöl, Mandarinenöl, Kiefernöl, Sonnenblumenöl,
- hydrogenierte Pflanzenöle wie hydrogeniertes Palmöl, hydrogeniertes Rapsöl, hydrogeniertes Sojabohnenöl,
- tierische Öle wie Schweinefettöl, Fischöle,
- Dialkylamide mittel bis langkettiger Fettsäuren z. B. Hallcomide sowie
- Pflanzenölester wie Rapsölmethylester.

Geeignete Verdicker sind Verbindungen, die der Formulierung ein pseudoplastisches Fließverhalten verleihen, d.h. hohe Viskosität im Ruhezustand und niedrige Viskosität im bewegten Zustand. Hier sind beispielsweise Polysaccharide bzw. organische Schichtmineralien wie Xanthan Gum^{®} (Kelzan^{®} der Fa. Kelco), Rhodopol^{®} 23 (Rhone Poulenc) oder Veegum^{®} (Firma R. T. Vanderbilt) oder Attaclay^{®} (Firma Engelhardt) zu nennen, wobei Xanthan-Gum^{®} bevorzugt verwendet wird.

Als für die erfindungsgemäßen Dispersionen geeignete Antischaummittel kommen beispielsweise Silikonemulsionen (wie z. B. Silikon^{®} SRE, Firma Wacker oder Rhodorsil^{®} der Firma Rhodia), langkettige Alkohole, Fettsäuren, fluororganische Verbindungen und deren Gemische in Betracht.

Bakterizide können zur Stabilisierung den erfindungsgemäßen Zusammensetzungen gegen Befall mit Mikroorganismen zugesetzt werden. Geeignete Bakterizide sind beispielsweise Proxel^{®} der Fa. ICI oder Acticide^{®} RS der Fa. Thor Chemie und Kathon^{®} MK der Firma Rohm & Haas.

Geeignete Frostschutzmittel sind organische Polyole, z. B. Ethylenglycol, Propylenglycol oder Glycerin. Diese werden üblicherweise in Mengen von nicht mehr als 10 Gew.-%, bezogen auf das Gesamtgewicht der Wirkstoffzusammensetzung eingesetzt um den gewünschten Gehalt an flüchtigen Verbindungen nicht zu überschreiten. In einer Ausführungsform der Erfindung beträgt der Anteil hiervon verschiedener flüchtiger organischer Verbindungen vorzugsweise nicht mehr als 1 Gew.-%, insbesondere nicht mehr als 1000 ppm.

Gegebenenfalls können die erfindungsgemäßen Wirkstoffzusammensetzungen 1 bis 5 Gew.-% Puffer bezogen auf die Gesamtmenge der hergestellten Formulierung zur pH-Wert Regulation enthalten, wobei sich die Menge und Art des eingesetzten Puffers nach den chemischen Eigenschaften des Wirkstoffes bzw. der Wirkstoffe richtet. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

Die folgenden Beispiele zur Herstellung und Verwendung der erfindungsgemäß zu verwendenden hyperverzweigten Polymere veranschaulichen die Erfindung, ohne sie jedoch in irgendeiner Weise zu beschränken.

### I. Herstellung hyperverzweigter Polymere

### Beispiel 1: Herstellung eines hyperverzweigten Polyharnstoffs

In einem Reaktionsgefäß, das mit Rührer, Innenthermometer und Stickstoffeinleitungsrohr versehen war, wurden unter Begasung mit trockenem Stickstoff 58,5 g wasserfreies n-Butanol vorgelegt und der Reaktionsansatz auf 75 °C erwärmt. Dann wurde innerhalb von 2,5 h 50 g eines Polyisocyanats auf Basis des Isocyanurats von Hexamethylendiisocyanat (Basonat® HI 100, mittlere NCO-Funktionalität ca. 3,7, mittlere Molmasse ca. 610 g/mol, Fa. BASF Aktiengesellschaft) so zugegeben, dass die Temperatur der Reaktionsmischung 80 °C nicht überschritt. Nach Zugabe des Polyisocyanats wurde noch 2 h bei 75 °C gerührt. Anschließend gab man 0,1 g Kaliumhydroxid (gelöst in 1,5 ml n-Butanol), 80,6 g Polyetheramin (Jeffamine® M-1000, monofunktionelles Aminogruppen-terminiertes Polyetherol, mittlere Molmasse ca. 1000 g/mol, Hansman Corp.) und 13,7 g Isophorondiamin zu, rührte weitere 10 min. bei 75 °C, erwärmte das Reaktionsgemisch anschließend auf 150 °C und rührte weitere 3,5 h bei dieser Temperatur. Anschließend ließ man das Reaktionsgemisch auf Raumtemperatur abkühlen, wobei ein wasserlösliches Produkt erhalten wurde. Der hyperverzweigte Polyharnstoff wurde per Gelpermeationschromatographie mit einem Refraktometer als Detektor analysiert. Als mobile Phase wurde Hexafluorisopropanol verwendet, als Standard zur Bestimmung des Molekulargewichts diente Polymethylmethacrylat (PMMA). Dabei wurde ein zahlenmittleres Molekulargewicht Mₙ von 2900 g/mol und ein gewichtsmittleres Molekulargewicht M_{w} von 32900 g/mol erhalten. Bei der Bestimmung des Schmelzpunkts mittels Differential Scanning Calorimetrie (DSC) wies das Produkt einen Schmelzpunkt von 31,4 °C auf.

### Beispiel 2: Herstellung eines hyperverzweigten Polyharnstoffs

In einem 3-Halskolben, der mit Rührer, Rückflusskühler und Innenthermometer ausgestattet war, wurden 60 g Tris(aminoethyl)amin, 36,2 g N,N'-Dimethylharnstoff und 0,1 g Kaliumcarbonat vorgelegt, auf 130 °C erwärmt und bei dieser Temperatur 7,5 h gerührt, wobei eine Gasentwicklung stattfand. Nach Abklingen der Gasentwicklung wurde das Reaktionsgemisch auf 140 °C aufgeheizt, weitere 2 h gerührt und danach auf Raumtemperatur abgekühlt. Es wurde ein wasserlösliches Produkt erhalten, welches wie in Beispiel 1 beschrieben analysiert wurde: Glasübergangstemperatur (Tg): -28 °C, Mₙ = 3100, M_{w} = 5100.

### Beispiel 3: Herstellung eines hyperverzweigten Polyamids

In einem für das Arbeiten unter Vakuum ausgerüsteten 3-Halskolben wurden 1380 g Adipinsäure durch Erhitzen auf 150 °C geschmolzen. Im Stickstoffstrom gab man bei dieser Temperatur 812 g Diethylentriamin tropfenweise innerhalb einer Stunde zu und ließ unter vermindertem Druck von 200 mbar bei 130 °C weiter reagieren, um das bei der Polykondensation gebildete Wasser abzutrennen. Das gebildete Wasser wurde mittels einer für die azeotrope Destillation vorgesehenen Apparatur gesammelt. Sobald ein rascher Anstieg der Viskosität des Reaktionsgemischs beobachtet wurde, d. h. vor Erreichen des Gelpunkts (etwa 4 h), wurde die Reaktion abgebrochen. Eine Bestimmung des Säurewerts des hyperverzweigten Prepolymers nach DIN 53402 ergab einen Wert von 212 mg KOH/g. Zu dem Prepolymer gab man 538 g Diethylentriamin und ließ weitere 8 h bei 130 °C und 200 mbar reagieren. Nach Abkühlen auf Raumtemperatur wurde ein hyperverzweigtes Polyamid erhalten. Eine Analyse wie in Beispiel 1 beschrieben ergab ein zahlenmittleres Molekulargewicht Mₙ von 3200 g/mol und ein gewichtsmittleres Molekulargewicht M_{w} von 6000 g/mol.

### Beispiel 4: Herstellung eines hyperverzweigten Polyesteramids

In einem für das Arbeiten unter Inertgas und Vakuum ausgestatteten Rundkolben mischte man 828 g Diethanolamin und 1380 g Adipinsäure unter Erwärmen auf 130 °C im Stickstoffstrom und ließ anschließend 2 h in Gegenwart von 2,25 g Dibutylzinnoxid als Katalysator bei 135 °C und unter vermindertem Druck von 300 mbar, um das bei der Polykondensation gebildete Wasser abzutrennen, reagieren. Sobald ein schneller Anstieg der Viskosität beobachtet wurde, bestimmte man den Säurewert (170 mg KOH/g) und gab 445 g Diethanolamin zu dem Prepolymer. Nach weiteren 3 h Reaktionsdauer unter Vakuum bei 135 °C wurde ein wasserlösliches hyperverzweigtes Polyesteramid erhalten, welches wie in Beispiel 1 beschrieben analysiert wurde. Das Produkt wies ein zahlenmittleres Molekulargewicht Mₙ von 3300 g/mol und ein gewichtsmittleres Molekulargewicht M_{w} von 11300 g/mol auf.

### Beispiel 5: Herstellung eines hyperverzweigten Polyesteramins

### 1. Umsetzung von Butylacrylat und Diethanolamin nach Art einer Michael-Addition

In einem 4 I-Vierhalskolben, der mit einer Vorrichtung für das Arbeiten unter Stickstoff ausgestattet war, wurden unter Stickstoffatmosphäre 1744 g Diethanolamin tropfenweise zu 1800 g n-Butylacrylat gegeben und das Reaktionsgemisch 2 h bei Raumtemperatur gerührt.

### 2. Polykondensation

Zu dem in Stufe 1 erhaltenen Reaktionsgemisch gab man 7,15 g Dibutylzinnoxid und erwärmte auf 135 °C, wobei die Reaktion zur Entfernung des bei der Polykondensationsreaktion gebildeten Methanols bei einem verminderten Druck von 200 mbar durchgeführt wurde. Nach 25 h wurde die Reaktion durch Kühlen des Reaktionsgemischs auf Raumtemperatur abgebrochen. Das erhaltene hyperverzweigte Polyesteramin wurde wie in Beispiel 1 beschrieben analysiert. Es wies ein zahlenmittleres Molekulargewicht Mₙ von 3100 g/mol und ein gewichtsmittleres Molekulargewicht M_{w} von 7600 g/mol auf.

### Beispiel 6: Herstellung eines hyperverzweigten Polyamids

In einem 3-Halskolben, der für das Arbeiten unter Stickstoff und Vakuum ausgestattet war, wurden 100 g Adipinsäure durch Erhitzen auf 150 °C geschmolzen. Dann gab man im Stickstoffstrom tropfenweise 14 g Diethylentriamin im Verlauf von 15 min. zu und ließ bei 120 °C weiter reagieren, wobei zur Abtrennung des bei der Polykondensation gebildeten Wassers ein verminderter Druck von 60 mbar eingesetzt wurde. Das Wasser wurde in einer für die azeotrope Destillation geeigneten Apparatur gesammelt. Sobald ein starker Anstieg der Viskosität zu beobachten war, d. h. vor Erreichen des Gelpunkts (etwa 6 h), wurde der Säurewert des Prepolymers nach DIN 53402 bestimmt, wobei ein Wert von 521 mg KOH/g erhalten wurde. Man gab 95,8 g Diethylentriamin zu und ließ weitere 10 h bei 120 °C und 60 mbar reagieren. Anschließend wurde das Reaktionsgemisch auf Raumtemperatur abgekühlt. Das erhaltene hyperverzweigte Polyamid wurde wie in Beispiel 1 beschrieben analysiert. Es wurde ein zahlenmittleres Molekulargewicht Mₙ von 4000 g/mol und ein gewichtsmittleres Molekulargewicht M_{w} von 6400 g/mol erhalten.

### 11. Anwendungstechnische Eigenschaften

### Allgemeine Arbeitsvorschrift 1:

Es wurden jeweils 1 bzw. 10 gew.-%ige Lösungen der zu bewertenden hyperverzweigten Polymere in N,N-Dimethylformamid (DMF) sowie 0,1 gew.-%ige Lösungen der Wirkstoffe ebenfalls in DMF bereitgestellt. Die Herstellung von Wirkstoffzusammensetzungen erfolgte durch Abmischung in einem Tecan-Pipettierroboter unter Verwendung von 1 ml Flachbodenglasgefäßen der Fa. HJ-Bioanalytik (96er Platten im Deepwell-Format). Es wurden jeweils 50 µl Polymer und 500 µl Wirkstofflösung in ein Gefäß gegeben und anschließend das Lösungsmittel durch 24-stündiges Trocknen im Vakuumtrockenschrank bei 70 °C und einem Druck unterhalb von 10 mbar entfernt. Anschließend wurden die Proben durch Zugabe von 500 µl Pufferlösung (Phosphatpuffer pH 6,8, 23,05 g Kaliumdihydrogenphosphat, 23,30 g di-Natriumhydrogenphosphat, VE-Wasser ad 5000 ml) und anschließendes zweistündiges Schütteln mittels eines HP-MTP-Schüttlers redispergiert. Die Beurteilung erfolgte durch Messung der Teilchengröße mittels Diffusion Light Scatttering nach einer Ruhephase von 2 h.

### Bewertung:

1 = ungenügende Redispergierung, Bodensatz
2 = keine eindeutige Beurteilung möglich
3 = vollständige Redispergierung mit Trübung (schwach bis milchig trüb)
4 = klare Lösung

### Allgemeine Arbeitsvorschrift 2:

0,5 g des gewählten Polymers und 0,1 g einer in Wasser zu lösenden Verbindung wurden in etwa 20 ml N,N-Dimethylformamid (DMF) gelöst. Das Gemisch wurde gerührt und anschließend von DMF befreit. Man erhielt eine feste Dispersion der gewählten, in Lösung zu bringenden Verbindung mit dem gewählten Copolymer. Die feste Dispersion wurde in 100 ml Wasser (gepuffert auf pH 6,8) gegeben und das Gemisch 24 h gerührt. Nach Filtration erhielt man Lösungen, deren Gehalt an der in Lösung zu bringenden Verbindung mittels HPLC und UV-Detektor bestimmt wurde. Tabelle 1 stellt die Literaturwerte für Wasserlöslichkeiten der gewählten Verbindungen sowie die Wellenlänge der UV-spektroskopischen Messung zusammen:

**Tabelle 1:**

| zu lösende Verbindung | Wasserlöslichkeit (ohne Polymer) [mg/l] | Wellenlänge der UV-Messung |
|---|---|---|
| Uvinul® T 150 | 0,007 | 308 nm |
| Cinidonethyl | 0,057 | 228 nm |
| Pyren | 0,13 | 330 nm |
| CI Solvent Red® | < 0,001 | 570 nm |
| Carbamazepin | 120 | 286 nm |
| Piroxicam | 200 | 270 nm |

### Allgemeine Arbeitsvorschrift 3:

In einem Becherglas wurden ca. 2 g Polymer eingewogen. Anschließend wurde dem Ansatz jeweils 0,2 g Piroxicam oder 0,3 g Carbamazepin zugewogen, um eine übersättigte Lösung zu erhalten. Anschließend wurden man 20 g Phosphatpuffer pH 7,0 zugegeben. Nach Filtration erhielt man Lösungen, deren Gehalt an der in Lösung zu bringenden Verbindung UV-spektroskopisch bestimmt wurde.

Die Literaturwerte für Wasserlöslichkeiten der gewählten Verbindungen sowie die Wellenlänge der UV-spektroskopischen Messung sind in Tabelle 2 zusammengestellt.

**Tabelle 2:**

| zu lösende Verbindung | Wasserlöslichkeit (ohne Polymer) [mg/l] | Wellenlänge der UV-Messung |
|---|---|---|
| Carbamazepin | 140 | 286 |
| Piroxicam | 420 | 356 |

### Beispiel 7:

Bestimmung der Eigenschaften wässriger Wirkstoffzusammensetzungen nach der allgemeinen Arbeitsvorschrift 1. Eingesetzt wurden Bentazone bei einem Polymer-Wirkstoff-Verhältnis von 1:1 und Metazachlor bei einem Polymer-Wirkstoff-Verhältnis von 10:1. Die Ergebnisse sind in Tabelle 3 zusammengestellt.

**Tabelle 3:**

| Polymer aus Beispiel Nr. | zu lösende Verbindung | Bewertung |
|---|---|---|
| 1 | Bentazone | 4 |
| 1 | Metazachlor | 3 |
| 6 | Bentazone | 4 |
| 6 | Metazachlor | 3 |

### Beispiel 8:

Bestimmung der Eigenschaften wässriger Wirkstoffzusammensetzungen nach der allgemeinen Arbeitsvorschrift 2. Die Ergebnisse sind in Tabelle 4 zusammengestellt.

**Tabelle 4:**

| zu lösende Verbindung | Löslichkeit [mg/l] in Gegenwart von | | |
|---|---|---|---|
| | Polymer 1 | Polymer 4 | Polymer 5 |
| Uvinul® T 150 | 122 | nb | nb |
| Cinidonethyl | 97 | nb | nb |
| CI Solvent Red® | 30 | nb | nb |
| Fipronil | 448 | nb | nb |
| Carbamazepin | 181 | 336 | 190 |
| Piroxicam | 1020 | nb | 1110 |
| Pyren | 63 | nb | nb |

### Beispiel 9:

Bestimmung der Eigenschaften wässriger Wirkstoffzusammensetzungen nach der allgemeinen Arbeitsvorschrift 3. Die Ergebnisse sind in Tabelle 5 zusammengestellt.

**Tabelle 5:**

| zu lösende Verbindung | Löslichkeit [mg/l] in Gegenwart von | |
|---|---|---|
| | Polymer 1 | Polymer 3 |
| Piroxicam | 9200 | 5300 |

## Patentansprüche

1. Wirk- oder Effektstoffzusammensetzung für die Kosmetik, enthaltend
A) wenigstens ein stickstoffatomhaltiges hyperverzweigtes Polymer, und
B) wenigstens einen Wirk- oder Effektstoff, der in Wasser bei 25 °C und 1013 mbar eine Löslichkeit unterhalb 10 g/l aufweist.

2. Zusammensetzung nach Anspruch 1, wobei das Polymer A) ausgewählt ist unter molekular und strukturell uneinheitlichen Polymeren.

3. Zusammensetzung nach einem der Ansprüche 1 oder 2, wobei das Polymer A) ausgewählt ist unter Polyurethanen, Polyharnstoffen, Polyamiden, Polyesteramiden, Polyesteraminen und Mischungen davon.

4. Wässrige Zusammensetzung nach einem der Ansprüche 1 bis 3, umfassend ein wässriges Medium als kontinuierliche Phase und wenigstens einen in der kontinuierlichen Phase solubilisierten oder dispergierten Wirkstoff und/oder Effektstoff B).

5. Zusammensetzung nach Anspruch 4, wobei die in der wässrigen Phase enthaltenden Wirk- und Effektstoffe Aggregate oder Partikel bilden, deren mittlere Teilchengröße, bestimmt mittels dynamischer Lichtstreuung, einen Wert von 300 nm nicht überschreiten.

6. Zusammensetzung in Form eines in wässrigen Medien redispergierbaren Feststoffs, erhältlich durch Trocknen einer wässrigen Wirk- oder Effektstoffzusammensetzung nach einem der Ansprüche 4 oder 5.

7. Zusammensetzung nach einem der vorhergehenden Ansprüche, enthaltend wenigstens einen Wirkstoff und/oder Effektstoff B) und wenigstens ein Polymer A) in einem Gewichtsverhältnis von 1:10 bis 3:1.

8. Zusammensetzung nach einem der vorhergehenden Ansprüche, mit einem Gehalt an flüchtigen organischen Verbindungen von weniger als 10 Gew.-%, bezogen auf das Gesamtgewicht der Zusammensetzung.

9. Verwendung wenigstens eines stickstoffatomhaltigen hyperverzweigten Polymers wie in einem der Ansprüche 1 bis 3 definiert, als Solubilisator zur Herstellung wässriger Formulierungen von Wirkstoffen und Effektstoffen, die in Wasser bei 25 °C/1013 mbar eine Löslichkeit unterhalb 10 g/l aufweisen.

10. Verfahren zur Herstellung einer wässrigen Wirk- oder Effektstoffzusammensetzung, wie in einem der Ansprüche 1 bis 8 definiert, umfassend:
i) Herstellung einer homogenen, nichtwässrigen Mischung, bestehend aus wenigstens einem stickstoffatomhaltigen hyperverzweigten Polymer und wenigstens einem Wirkstoff und/oder Effektstoff, und
ii) Dispergieren der so erhaltenen Mischung mit Wasser.

11. Verfahren zur Herstellung einer wässrigen Wirk- oder Effektstoffzusammensetzung, wie in einem der Ansprüche 1 bis 8 definiert, umfassend:
i) Herstellung einer Lösung von Wirkstoff und/oder Effektstoff und gegebenenfalls wenigstens einem stickstoffatomhaltigen hyperverzweigten Polymer in einem organischen Lösungsmittel, das einen Siedepunkt unter dem von Wasser aufweist und
ii) Vermischen der Lösung des Wirkstoffs und/oder Effektstoffs mit Wasser oder einer wässrigen Lösung des hyperverzweigten Polymers und
iii) Entfernen des organischen Lösungsmittels.

12. Verfahren zur Herstellung einer wässrigen Wirk- oder Effektstoffzusammensetzung, wie in einem der Ansprüche 1 bis 8 definiert, umfassend das Einarbeiten des Wirkstoffs und/oder des Effektstoffs in eine wässrige Lösung des stickstoffatomhaltigen hyperverzweigten Polymers bei einer Temperatur oberhalb der Schmelztemperatur des Wirkstoffs.

13. Kosmetisches Mittel, enthaltend
A) wenigstens ein stickstoffatomhaltiges, hyperverzweigtes Polymer, wie in einem der Ansprüche 1 bis 3 definiert,
B) wenigstens einen kosmetisch akzeptablen Wirk- oder Effektstoff, der in Wasser bei 25 °C und 1013 mbar eine Löslichkeit unterhalb 10 g/l aufweist, und
C) gegebenenfalls wenigstens einen weiteren, von B) verschiedenen kosmetisch akzeptablen Wirk- oder Hilfsstoff.

14. Mittel nach Anspruch 13, wobei die Komponenten B) und C) nach Maßgabe ihrer Löslichkeit ausgewählt sind unter kosmetisch akzeptablen Trägern, Emulgatoren, Tensiden, Konservierungsmitteln, Parfümölen, Verdickern, Haarpolymeren, Haar-und Hautconditionern, wasserlöslichen oder dispergierbaren siliconhaltigen Polymeren, Bleichmitteln, Gelbildnern, Pflegemitteln, Färbemitteln, Tönungsmitteln, Bräunungsmitteln, Farbstoffen, Pigmenten, Antischuppenmitteln, Lichtschutzmitteln, desodorierenden Wirkstoffen, Vitaminen, Pflanzenextrakten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

15. Mittel nach einem der Ansprüche 13 oder 14, enthaltend wenigstens einen schwerlöslichen UV-Absorber.
